# EUROPEAN PATENT APPLICATION

(11) **EP 4 454 541 A1**
(43) Date of publication of application: **30.10.2024**
(21) Application number: 22910965.7
(22) Date of filing: 09.12.2022
(51) Int. Cl.: A61B 1/018, A61B 1/00

(54) **OVERSHEATH, ENDOSCOPIC TREATMENT TOOL DEVICE, AND ENDOSCOPIC INSTRUMENT**

(30) Priority: 24.12.2021 JP 2021211328
(71) Applicant: FUJIFILM Corporation, Tokyo 106-8620 (JP)
(72) Inventor: ISHIKAWA, Ryo, Ashigarakami-gun, Kanagawa 258-8538 (JP); IWAKI, Takuro, Ashigarakami-gun, Kanagawa 258-8538 (JP)
(74) Representative: HGF
(86) International application number: PCT/JP2022/045538
(87) International publication number: WO 2023/120255

(57) **Abstract**

Provided are an oversheath, an endoscopic treatment tool device, and an endoscopic instrument which make it possible to easily perform a treatment tool operation that can be performed by either a doctor or an assistant who operates an endoscope with a small number of people and that can correspond to a plurality of types of endoscopic treatment tools.

An oversheath (22) is the oversheath (22) into which a flexible sheath (23) of a treatment tool body (21) is inserted and that is inserted into a forceps channel in cooperation with the treatment tool body (21). The oversheath (22) includes a first tubular part (41) including an outer peripheral side sliding part (41A) that slides on a forceps port as an introduction port into the forceps channel, and a second tubular part (42) provided at a base end part of the first tubular part (41) and including an inner peripheral side sliding part (42A) that allows the endoscopic treatment tool body (21) to move forward and backward with respect to the forceps channel (15).

## Description

### BACKGROUND OF THE INVENTION

### 1. Field of the Invention

The present invention relates to an oversheath and an endoscopic treatment tool device that are used by being inserted into a forceps channel of an endoscope, and an endoscopic instrument.

### 2. Description of the Related Art

In the medical field, an insertion part of an endoscope is inserted into a body of a subject to perform various treatments on an observation site in addition to observation of an inside of the body. Specifically, various treatment tools, such as forceps and an incision tool, are inserted into a forceps channel in the insertion part from a forceps port provided in an operating part of the endoscope and are led out from a forceps outlet being open at a distal end of the insertion part, and various treatments such as resection and collection of tissues of the observation site are performed.

JP4663345B describes an endoscopic treatment tool device comprising an endoscopic treatment tool having a flexible sheath, and an attachment part for attaching the flexible sheath to an operating part of an endoscope. The flexible sheath is inserted into a body cavity through a forceps channel of the endoscope. The endoscopic treatment tool includes the flexible sheath, a flexible operating wire, a treatment part provided at a distal end of the operating wire, and a hard pipe coupled to a base end of the operating wire. The operating wire and the hard pipe are supported to be movable forward and backward with respect to the flexible sheath. The treatment part is a snare wire that is contracted in a case of being accommodated in the flexible sheath and is expanded in a case of protruding from a distal end of the flexible sheath. As a result, an operation of expanding the treatment part by allowing the treatment part to protrude from the distal end of the flexible sheath inserted into the body cavity can be performed in the vicinity of the operating part of the endoscope.

### SUMMARY OF THE INVENTION

In the medical field, it is desired to take further preventive measures against infectious diseases. Therefore, it is required to reduce a risk of infection by performing various treatments using the endoscope and the treatment tool with as few people as possible. In addition, due to a problem, such as a shortage of medical personnel or an increase in burden of medical expenses, it is desired to perform the treatment with a small number of people. In addition, in a case in which an assistant who operates the treatment tool exists in addition to a doctor who operates the endoscope, in order to smoothly perform the treatment, it is necessary to have good cooperation between the doctor and the assistant, and there is a risk that a burden on the doctor is increased.

However, in the endoscopic treatment tool described in JP4663345B, in a case in which the doctor operates the endoscope, the operation of the endoscopic treatment tool that can be performed by the doctor is only an operation of allowing the treatment tool to move forward and backward with respect to the forceps channel. That is, for operations other than the operation of allowing the treatment tool to move forward and backward with respect to the forceps channel, for example, an operation of expanding or contracting the treatment part or an operation of rotating the treatment tool about a central axis with respect to the forceps channel, the assistance from the assistant is required, and the doctor needs cooperation in an operation, such as issuing an instruction to the assistant. Moreover, the number of people who perform the treatment cannot be reduced.

In addition, in the endoscopic treatment tool device described in JP4663345B, a dedicated component, such as the hard pipe or the attachment part, is incorporated, and the device is not configured to correspond to various endoscopic treatment tools. In the medical field, in a case in which various treatments are performed, it is necessary to use various endoscopic treatment tools.

An object of the present invention is to provide an oversheath, an endoscopic treatment tool device, and an endoscopic instrument which make it possible to easily perform a treatment tool operation that can be performed by either a doctor or an assistant who operates an endoscope with a small number of people and that can correspond to a plurality of types of endoscopic treatment tools.

An aspect of the present invention relates to an oversheath into which a flexible sheath of an endoscopic treatment tool is inserted and that is inserted into a forceps channel of an endoscope in cooperation with the endoscopic treatment tool, the oversheath comprising: a first tubular part; and a second tubular part. The first tubular part includes an outer peripheral side sliding part that slides on an inner peripheral surface of a forceps port as an introduction port into the forceps channel. The second tubular part is provided at a base end part of the first tubular part and includes an inner peripheral side sliding part that allows the endoscopic treatment tool to move forward and backward with respect to the forceps channel.

It is preferable that the oversheath further comprises an outer sheath in which the first tubular part and the second tubular part are provided integrally with each other; and an inner sheath in which a fitting part that is externally fitted to the endoscopic treatment tool, and a third tubular part that slides on the inner peripheral side sliding part and covers at least a part of the flexible sheath are provided integrally with each other.

It is preferable that the oversheath further comprises an attachment/detachment member provided integrally with the outer sheath and engaging with a forceps valve mounted on the forceps channel, in which, in a case in which the attachment/detachment member engages with the forceps valve, the outer sheath is attached to the endoscope via the attachment/detachment member. It is preferable that the attachment/detachment member includes a first engaging part that engages with the forceps valve, and a second engaging part that engages with a part of the endoscope. It is preferable that the attachment/detachment member is disposed in a direction in which an attachment direction to the forceps valve is orthogonal to an axial direction of the outer sheath or in a direction inclined from the direction orthogonal to the axial direction.

It is preferable that the endoscopic treatment tool is movable forward and backward and rotatable with respect to the oversheath within a range in which the third tubular part and the inner peripheral side sliding part slide.

It is preferable that the fitting part is fitted to the flexible sheath of the endoscopic treatment tool or an operating part installed consecutively with the flexible sheath. It is preferable that the third tubular part has a dimension in an axial direction longer than a range in which the endoscopic treatment tool moves forward and backward with respect to the forceps channel.

It is preferable that the outer sheath includes a seal member that fills a gap between the outer sheath and the inner sheath at a base end part of the second tubular part. It is preferable that the first tubular part has a double-tube structure in which a length in an axial direction is expandable and contractible. It is preferable that a length of the first tubular part is shorter than a length of the flexible sheath.

It is preferable that the forceps channel includes a first inner diameter part located on a forceps port side, and a second inner diameter part located on a distal end part side of the endoscope with respect to the first inner diameter part and having a smaller inner diameter than the first inner diameter part, and an outer diameter of the first tubular part is smaller than the first inner diameter part and is larger than the second inner diameter part.

It is preferable that a first resistance force in a case of allowing the first tubular part to move forward and backward with respect to the forceps channel is larger than a second resistance force in a case of allowing the endoscopic treatment tool to move forward and backward with respect to the second tubular part.

Another aspect of the present invention relates to an endoscopic treatment tool device comprising: a treatment tool body including a tubular flexible sheath, an operating wire, and an operating part; and an oversheath having an insertion pipe line into which the flexible sheath is inserted and being inserted into a forceps channel of an endoscope in cooperation with the treatment tool body, in which the oversheath includes an outer sheath in which a first tubular part and a second tubular part are provided integrally with each other, and an inner sheath in which a fitting part and a third tubular part are provided integrally with each other. The operating wire is inserted into the flexible sheath, and the operating part is provided at a base end part of the flexible sheath. The oversheath has an insertion pipe line into which the flexible sheath is inserted and is inserted into a forceps channel of an endoscope in cooperation with the treatment tool body. The first tubular part includes an outer peripheral side sliding part that slides on an inner peripheral surface of a forceps port as an introduction port into the forceps channel. The second tubular part is provided at a base end part of the first tubular part and includes an inner peripheral side sliding part that allows the treatment tool body to move forward and backward with respect to the forceps channel. The fitting part is externally fitted to the treatment tool body. The third tubular part slides on the inner peripheral side sliding part and covers at least a part of the flexible sheath. It is preferable that the endoscopic treatment tool device further comprises an attachment/detachment member provided integrally with the outer sheath and engaging with a forceps valve mounted on the forceps channel, in which, in a case in which the attachment/detachment member engages with the forceps valve, the outer sheath is attached to the endoscope via the attachment/detachment member.

Still another aspect of the present invention relates to an endoscopic instrument comprising: an attachment/detachment member, in which the attachment/detachment member includes a first engaging part that engages with the forceps valve, and a second engaging part that engages with a part of an endoscope, and in a case in which the attachment/detachment member engages with the forceps valve, the endoscopic instrument is attached to the endoscope via the attachment/detachment member. The attachment/detachment member engages with the forceps valve mounted on the forceps channel of the endoscope. It is preferable that the endoscopic instrument further comprises a treatment tool operating part that operates an endoscopic treatment tool, in which the attachment/detachment member is provided integrally with the treatment tool operating part. The treatment tool operating part operates the endoscopic instrument. It is preferable that the part of the endoscope engaged with the second engaging part is located on a proximal end side of the forceps valve. It is preferable that the endoscopic instrument is disposed at a position at which an axial direction intersects with an attachment/detachment direction of the attachment/detachment member. It is preferable that the part of the endoscope engaged with the second engaging part is a treatment tool introduction part that constitutes an operating part of the endoscope, and the endoscopic instrument is disposed in a direction along the treatment tool introduction part.

According to the present invention, it is possible to easily perform a treatment tool operation that can be performed by either a doctor or an assistant who operates an endoscope with a small number of people and that can correspond to a plurality of types of endoscopic treatment tools.

### BRIEF DESCRIPTION OF THE DRAWINGS

Fig. 1 is a schematic diagram showing an endoscopy using an endoscope and an endoscopic treatment tool device.
Fig. 2 is a front view of the endoscope and the endoscopic treatment tool device.
Fig. 3 is a cross-sectional view of main parts in the periphery of a forceps valve in the endoscope.
Fig. 4 is a front view of the endoscopic treatment tool device.
Figs. 5A and 5B are explanatory diagrams showing a state in which a snare wire is expanded and a state in which the snare wire is contracted, respectively.
Fig. 6 is a cross-sectional view of a main part of an oversheath.
Fig. 7 is an exploded perspective view showing a configuration of the oversheath.
Fig. 8 is an exploded perspective view showing a configuration of an outer sheath.
Figs. 9A and 9B are explanatory diagrams showing a state in which a first tubular part of the outer sheath is contracted and a state in which the first tubular part of the outer sheath is expanded.
Fig. 10 is an explanatory diagram showing a relationship between an inner diameter of a forceps channel and an outer diameter of the outer sheath.
Fig. 11 is a cross-sectional view of main parts in the periphery of an inner peripheral side sliding part and an attachment/detachment member.
Fig. 12 is a cross-sectional view of main parts in the periphery of a seal member and the attachment/detachment member in a state in which a third tubular part of an inner sheath is inserted into the inner peripheral side sliding part of the outer sheath.
Fig. 13 is a perspective view of the endoscopic treatment tool in a state in which the inner sheath is fitted, and the outer sheath.
Fig. 14 is a perspective view of a state in which the endoscopic treatment tool is inserted into the oversheath.
Fig. 15 is a perspective view of the periphery of the attachment/detachment member.
Fig. 16 is a perspective view showing a state before the endoscopic treatment tool device is attached to the endoscope.
Fig. 17 is an explanatory diagram showing an operation of performing a treatment using the endoscopic treatment tool device.
Fig. 18 is an explanatory diagram of treating a lesion portion by using the endoscopic treatment tool device, which shows a state (A) in which a position of a distal end part of a flexible sheath is aligned with a position of the lesion portion and a state (B) in which the snare wire is made to protrude.
Fig. 19 is an explanatory diagram of treating the lesion portion by using the endoscopic treatment tool device, which shows a state (A) in which the lesion portion is surrounded by the snare wire and a state (B) in which the lesion portion is resected.
Fig. 20 is a front view of an endoscopic treatment tool device according to a second embodiment.
Fig. 21 is a cross-sectional view of main parts in the periphery of an inner peripheral side sliding part and an attachment/detachment member in the endoscopic treatment tool device according to the second embodiment.
Fig. 22 is an explanatory diagram showing an operation of performing an endoscopy and a treatment in a first modification example.
Fig. 23 is an explanatory diagram showing an operation of performing an endoscopy and a treatment in a second modification example.
Figs. 24A and 24B are plan views showing a disposition of an outer sheath in a case in which an axial direction in a third modification example is not inclined and a case in which the axial direction is inclined, respectively.
Fig. 25 is a plan view showing a disposition of an outer sheath in a fourth modification example.
Fig. 26 is a perspective view of an endoscope and an endoscopic treatment tool device in a fifth modification example.
Fig. 27 is a perspective view of the endoscope and the endoscopic treatment tool device in the fifth modification example as viewed from another direction.
Fig. 28 is a plan view of the endoscopic treatment tool device in the fifth modification example.
Fig. 29 is a perspective view of an endoscope and an endoscopic instrument according to a third embodiment.
Fig. 30 is a perspective view of an endoscopic instrument according to the third embodiment.
Fig. 31 is a perspective view of an endoscope and an endoscopic instrument according to a fourth embodiment.
Fig. 32 is a perspective view of an endoscope distal end part according to the fourth embodiment.
Fig. 33 is an exploded perspective view of the endoscope distal end part according to the fourth embodiment.
Fig. 34 is an explanatory diagram showing an operation of the endoscopic instrument according to the fourth embodiment.

### DESCRIPTION OF THE PREFERRED EMBODIMENTS

### [First embodiment]

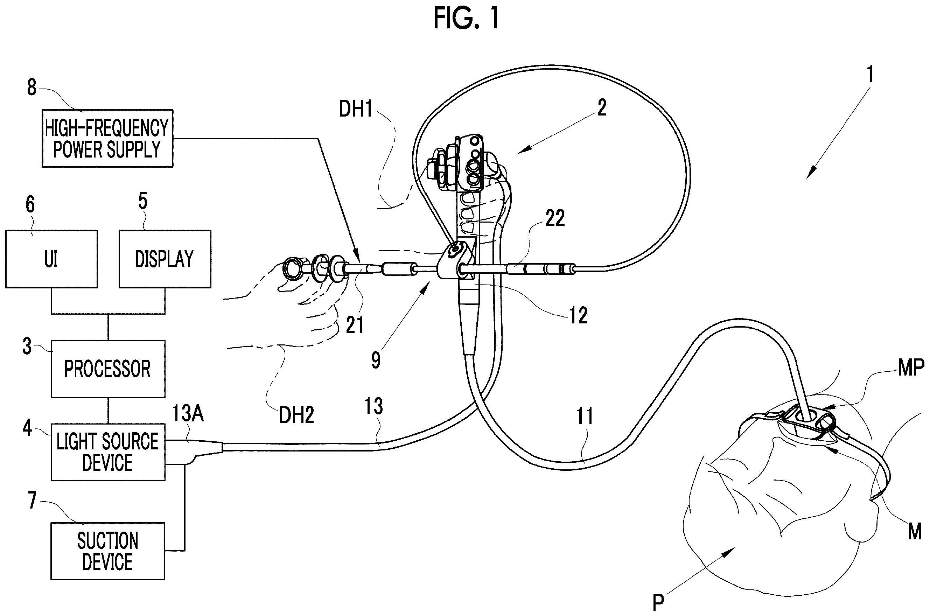

As shown in Fig. 1, an endoscope system 1 according to the embodiment of the present invention comprises an endoscope 2, a processor device 3, a light source device 4, a display 5, a user interface (UI) 6, a suction device 7, a high-frequency power supply 8, and an endoscopic treatment tool device 9. The endoscope 2 is, for example, an upper gastrointestinal tract endoscope for an esophagus, a stomach, or the like, and comprises an insertion part 11 inserted into an upper gastrointestinal tract of a patient P as a subject, an operating part 12 installed consecutively with a base end part of the insertion part 11, a universal cord 13 connected to the operating part 12.

The universal cord 13 is connected to an external device, such as the processor device 3 or the light source device 4, via a connector 13A. The processor device 3 is electrically connected to the display 5 and the UI 6. The UI 6 includes a keyboard, a mouse, a touch pad, a microphone, and the like, and receives an input operation of a doctor D who is an operator.

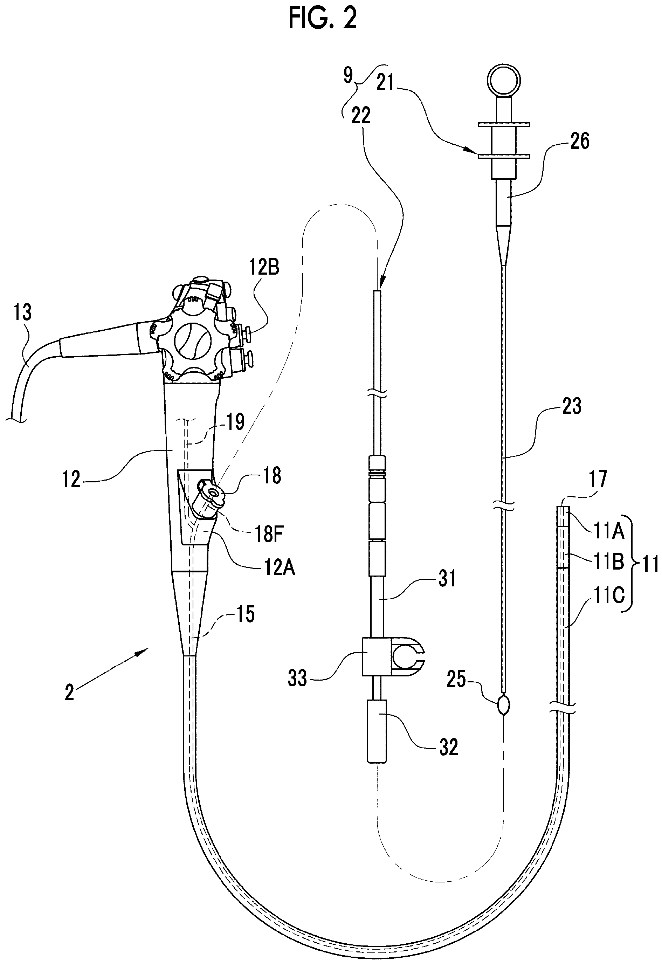

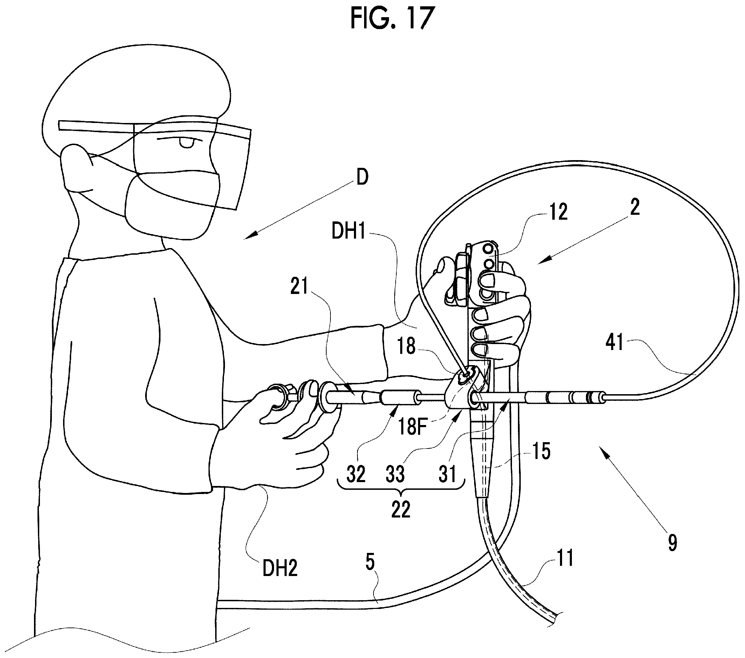

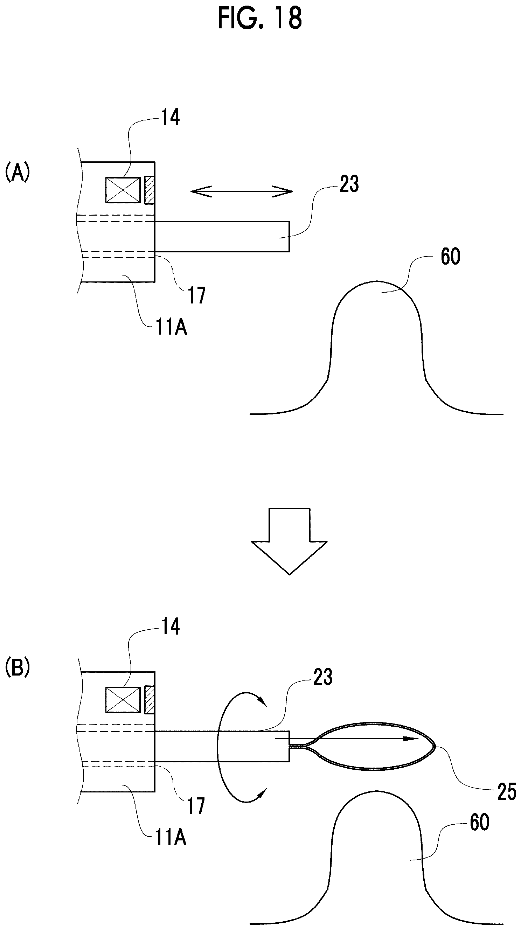

As shown in Fig. 2, the insertion part 11 consists of a distal end part 11A, a bendable part 11B, and a flexible tube part 11C having flexibility in this order from a distal end side to a base end side. An observation window or an illumination window is provided on a distal end surface of the distal end part 11A (not shown). An image sensor 14 (see Figs. 17 and 18) or the like is disposed behind the observation window, and a light guide (optical fiber) cable (not shown) is disposed behind the illumination window. A signal line or the light guide cable of the image sensor 14 is connected to the processor device 3 and the light source device 4 through the insertion part 11, the operating part 12, the universal cord 13, and the connector 13A. The processor device 3 performs image processing or the like on an endoscopic image captured by the image sensor 14 and displays the endoscopic image on the display 5. The bendable part 11B is installed consecutively with the distal end part 11A and is provided to be bendable.

In a case in which the insertion part 11 of the endoscope 2 is inserted from a mouth M of the patient P, an endoscopic mouthpiece MP is mounted on the mouth of the patient P. The endoscopic mouthpiece MP has a pipe line (not shown) into which the insertion part 11 is inserted. The endoscopic mouthpiece MP is mounted on the mouth M of the patient P by inserting a part of the endoscopic mouthpiece MP into the mouth M of the patient P and holding the inserted part by the patient P. As a result, the insertion part 11 can be introduced into a body through the pipe line.

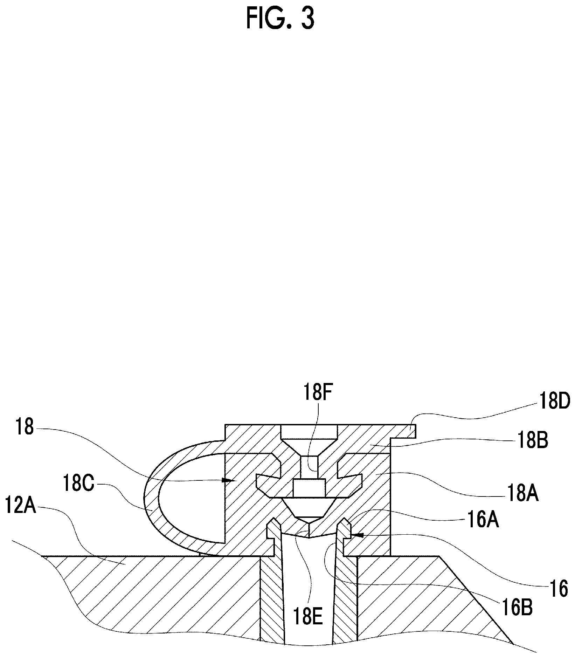

A forceps channel 15 into which a treatment tool body 21 and an oversheath 22, which will be described below, are inserted is disposed in the insertion part 11. A treatment tool introduction part 12A is integrally provided in the operating part 12. The treatment tool introduction part 12A is a protruding part that protrudes from an outer peripheral surface of the operating part 12. The treatment tool introduction part 12A comprises a socket 16 (see Fig. 3). The forceps channel 15 has one end connected to a forceps outlet 17, and the other end connected to the socket 16.

As shown in Fig. 3, the socket 16 has a flange part 16A on an outer peripheral surface. An inner peripheral surface 16B of the socket 16 is continuous with an inner peripheral surface of the forceps channel 15. A forceps valve 18 is mounted on the socket 16. The flange part 16A locks the forceps valve 18.

The forceps valve 18 is formed of an elastically deformable resin, a rubber member, or the like. The forceps valve 18 comprises, for example, a cylindrical valve body 18A, a cap 18B to be fitted to the valve body 18A, and an attachment arm 18C. The cap 18B has a grip part 18D that protrudes from an outer diameter. The attachment arm 18C couples the valve body 18A and the cap 18B. A slit valve 18E formed with a slit into which the treatment tool body 21 is inserted is provided in the valve body 18A. The cap 18B is provided with a treatment tool insertion hole 18F.

The treatment tool insertion hole 18F is located on the same axis as the inner peripheral surface 16B of the socket 16. As described above, the inner peripheral surface 16B of the socket 16 is continuous with the inner peripheral surface of the forceps channel 15, and thus the treatment tool insertion hole 18F serves as an introduction port into the forceps channel 15. The treatment tool insertion hole 18F in the present embodiment corresponds to a "forceps port" in the claims.

In addition, the forceps channel 15 is also used as a path for suctioning a content such as body fluid, such as blood, or an internal waste product, and a path for feeding a washing solution, such as water, from the forceps outlet 17. A suction channel 19 branching from the forceps channel 15 is disposed in the operating part 12. The suction channel 19 has one end connected to the forceps channel 15, and the other end connected to an operation button 12B provided in the operating part 12.

The operation button 12B comprises a suction valve (not shown) provided therein. The suction valve is connected to the suction channel 19 in the operating part 12, and is connected to the external suction device 7 via the pipe lines (not shown) disposed in the operating part 12, the universal cord 13, and the connector 13A. The suction device 7 is, for example, a suction pump that generates a negative pressure. In a case in which a pressing part of the operation button 12B is operated in a pressed manner, the suction channel 19 communicates with the pipe line of the suction device 7. As a result, the body fluid or the like can be sucked from the forceps outlet 17 of the insertion part 11 inserted into the subject or the like. By releasing the pressing operation of the pressing part, the communication between the suction channel 19 and the pipe line of the suction device 7 is interrupted, and the suction from the forceps outlet 17 can be stopped.

The high-frequency power supply 8 is connected to the endoscopic treatment tool device 9 via a cable (not shown), and allows a high-frequency current to flow through a snare wire 25, which will be described below, of the endoscopic treatment tool device 9. As a result, the lesion portion can be resected.

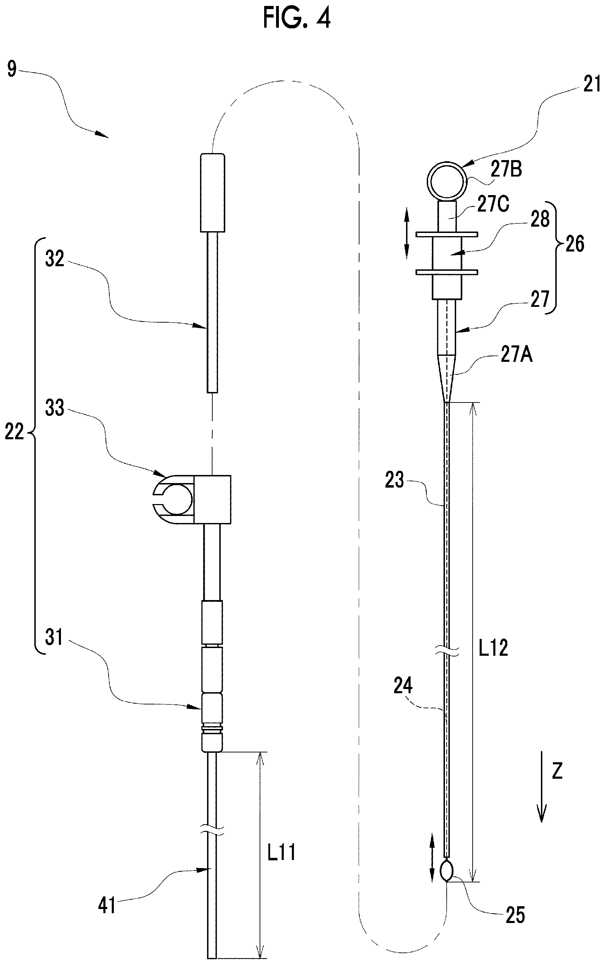

As shown in Fig. 4, the endoscopic treatment tool device 9 comprises the treatment tool body 21 and the oversheath 22. The treatment tool body 21 corresponds to an "endoscopic treatment tool" or a "treatment tool body" in the claims. The treatment tool body 21 is, for example, a high-frequency snare for resecting the lesion portion by allowing the high-frequency current to flow. The oversheath 22 is inserted into the forceps channel 15 in cooperation with the treatment tool body 21.

The treatment tool body 21 comprises a flexible sheath 23, an operating wire 24, the snare wire 25 as a treatment part, and an operating part 26. The flexible sheath 23 is a tubular sheath formed of a flexible material, for example, a soft resin, and is inserted into the forceps channel 15 of the endoscope 2 along with the oversheath 22. The operating wire 24 is provided integrally with the snare wire 25 and is inserted into the flexible sheath 23.

The operating part 26 comprises an operating part body 27 and a slider 28 that is slidingly supported by the operating part body 27. The operating part body 27 is installed consecutively with a base end part of the flexible sheath 23. A tapered part 27A of which an outer diameter gradually decreases from the base end side toward the distal end side is provided on a distal end part of the operating part body 27.

The operating part body 27 is provided with a finger hook part 27B and a cylindrical part 27C parallel to an insertion direction Z. The slider 28 engages with the cylindrical part 27C and slidingly moves along the cylindrical part 27C in an axial direction of the flexible sheath 23. In a case in which the patient P is treated, the thumb of the operator is hooked on the finger hook part 27B, and the index finger and the middle finger of the same operator are hooked on the slider 28. A base end of the operating wire 24 is fixed to the slider 28. Therefore, the operating wire 24 is operated in a push-pull manner in the flexible sheath 23 in the axial direction thereof is performed in response to the sliding movement of the slider 28.

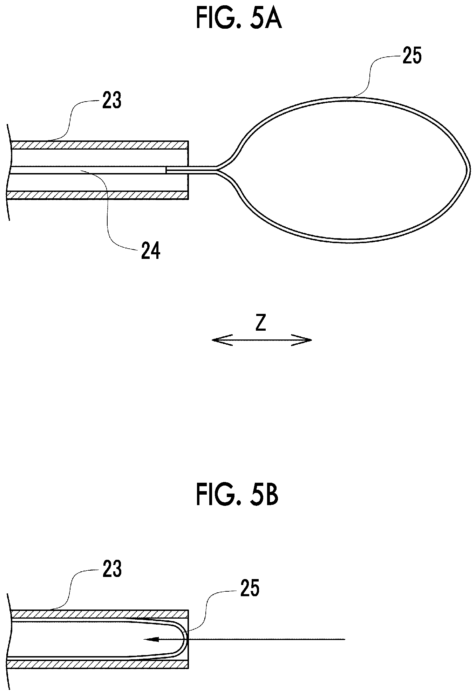

As shown in Fig. 5A, the snare wire 25 is expanded in a loop shape in a case of protruding from the distal end of the flexible sheath 23 in the insertion direction Z due to the operating wire 24 operated in a push-pull manner. As a result, the snare wire 25 can surround the lesion portion. On the other hand, as shown in Fig. 5B, in a case in which the snare wire 25 is accommodated in the flexible sheath 23 by the push-pull operation of the operating wire 24 in response to the sliding movement of the slider 28, the snare wire 25 is in a contracted state.

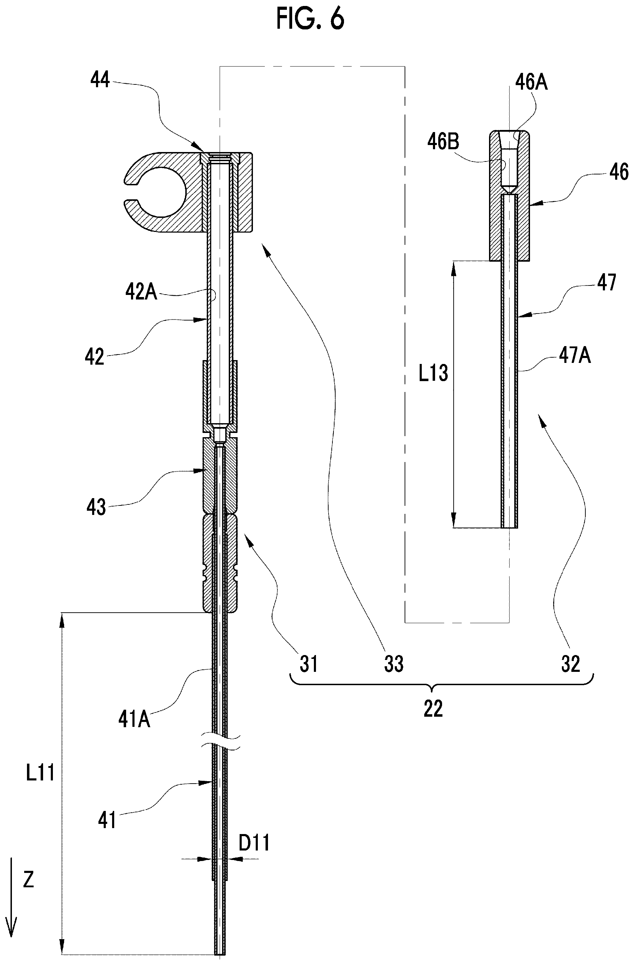

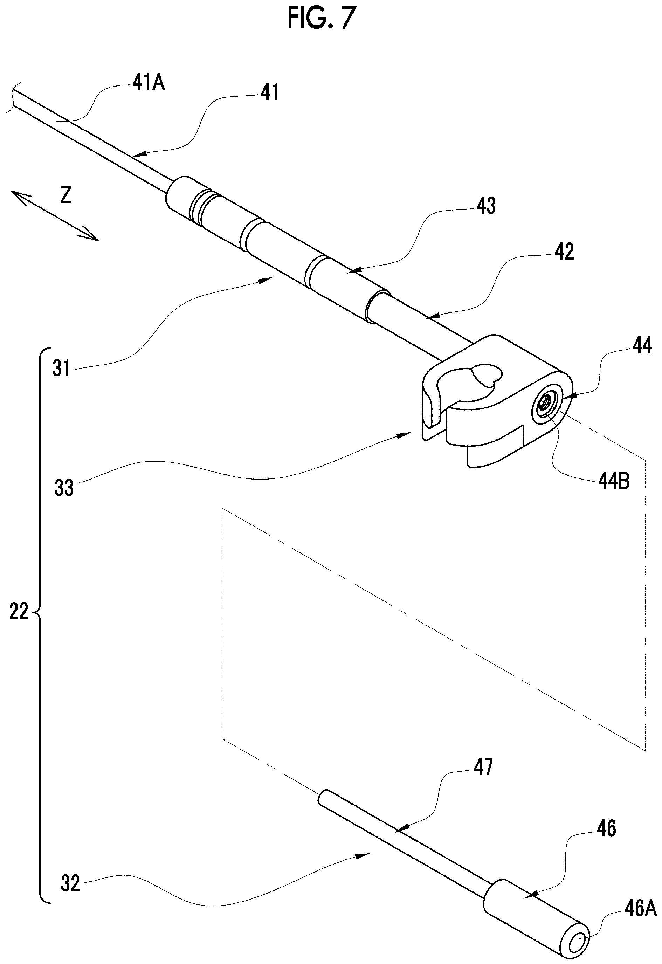

As shown in Figs. 6 and 7, the oversheath 22 comprises an outer sheath 31, an inner sheath 32, and an attachment/detachment member 33. In the outer sheath 31, a first tubular part 41, a second tubular part 42, a coupling member 43, and a seal member 44 are provided integrally with each other. The first tubular part 41, the second tubular part 42, the coupling member 43, and the seal member 44 are formed of, for example, a soft material, such as a soft resin.

In addition, a length L11 of the first tubular part 41 in the insertion direction Z is shorter than a length L12 (see Fig. 4) of the flexible sheath 23. As a result, in a case in which the flexible sheath 23 is inserted into the outer sheath 31, the distal end of the flexible sheath 23 protrudes from the distal end of the outer sheath 31. Therefore, in a case in which the operating wire 24 is operated in a push-pull manner, the snare wire 25 does not come into contact with the outer sheath 31, and the snare wire 25 can be brought into an expanded state.

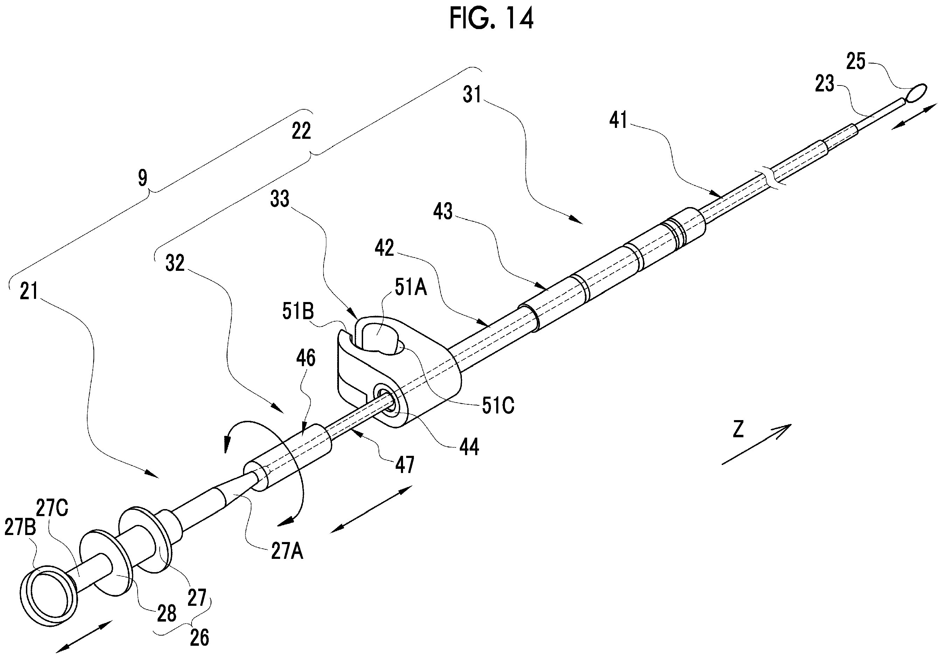

The first tubular part 41 is a flexible tubular sheath, and the flexible sheath 23 of the treatment tool body 21 is inserted into the first tubular part 41 (see Fig. 14). An outer peripheral surface of the first tubular part 41 is an outer peripheral side sliding part 41A. The outer peripheral side sliding part 41A is formed to match an inner peripheral surface of the treatment tool insertion hole 18F and has a slightly larger outer diameter than a hole diameter of the treatment tool insertion hole 18F. It should be noted that the term "slightly larger outer diameter" used herein refers to an outer diameter within a range in which the hole diameter of the treatment tool insertion hole 18F is expanded due to elastic deformation. As a result, the outer peripheral side sliding part 41A slides on the inner peripheral surface of the treatment tool insertion hole 18F.

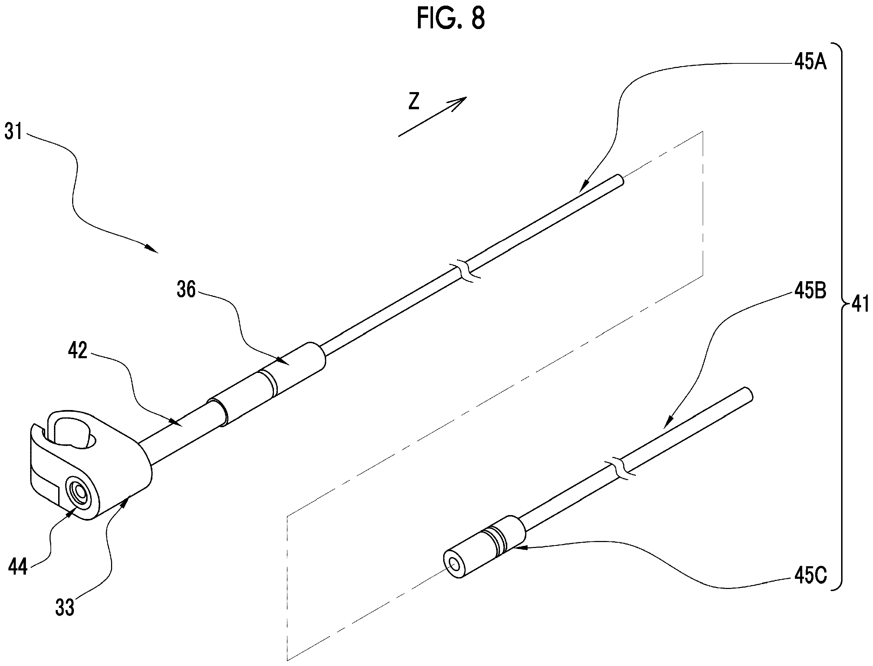

As shown in Fig. 8, the first tubular part 41 formed of a small-diameter tube 45A, a large-diameter tube 45B, and a grip member 45C. The large-diameter tube 45B is formed to have a length equal to or slightly shorter than a length of the small-diameter tube 45A in the axial direction, that is, the insertion direction Z.

The first tubular part 41 has a double-tube structure in which the length in the insertion direction Z is expandable and contractible. Specifically, the large-diameter tube 45B covers the small-diameter tube 45A and can move relative to the small-diameter tube 45A in the insertion direction Z. The grip member 45C is formed to have a larger outer diameter than the large-diameter tube 45B, and the large-diameter tube 45B is firmly attached to an inner peripheral surface thereof. The grip member 45C moves integrally with the large-diameter tube 45B.

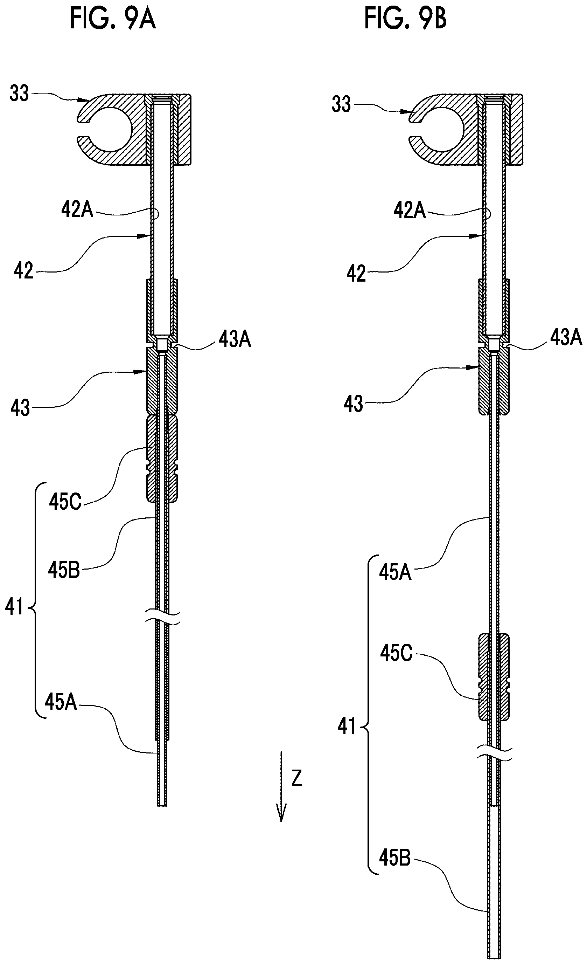

As shown in Fig. 9A, the first tubular part 41 covers the small-diameter tube 45A over the entire large-diameter tube 45B in a normal state. That is, the length of the first tubular part 41 in the insertion direction Z is substantially equal to the length of the small-diameter tube 45A in the insertion direction Z. On the other hand, as shown in Fig. 9B, in a case in which a user grips the grip member 42C and moves the large-diameter tube 45B to the distal end side, a part in which the large-diameter tube 45B and the small-diameter tube 45A overlap with each other is reduced. That is, the length of the first tubular part 41 in the insertion direction Z is longer than the length in the state shown in Fig. 9A. In a case in which the large-diameter tube 45B is moved to the base end side from the state shown in Fig. 9B, the length of the first tubular part 41 in the insertion direction Z is shortened. In this manner, the first tubular part 41 can be expanded and contracted.

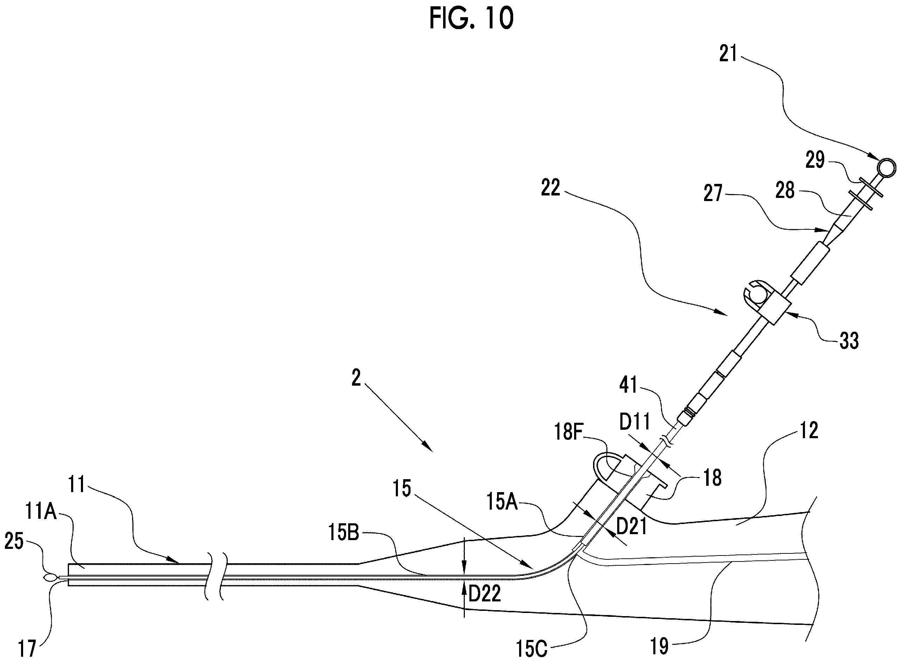

As shown in Fig. 10, the forceps channel 15 of the endoscope 2 includes a first inner diameter part 15A located on the forceps port side, that is, the treatment tool insertion hole 18F side, and a second inner diameter part 15B located on the distal end part 11A side with respect to the first inner diameter part 15A. An inner diameter D22 of the second inner diameter part 15B is smaller than an inner diameter D21 of the first inner diameter part 15A. It should be noted that Fig. 10 shows a state in which the attachment/detachment member 33 does not engage with the forceps valve 18 for convenience of description.

The forceps channel 15 includes a branch part 15C combined with the suction channel 19. The branch part 15C is formed of, for example, a hard material, such as a metal pipe. A part between the branch part 15C and the socket 16 (see Fig. 3) is the first inner diameter part 15A, and a part between the branch part 15C and the forceps outlet 17 is the second inner diameter part 15B. The first inner diameter part 15A and the first inner diameter part 15A are formed of, for example, a pipe made of a resin.

An outer diameter D11 (see Fig. 6) of the first tubular part 41 is smaller than the inner diameter D21 of the first inner diameter part 15A and is larger than the inner diameter D22 of the second inner diameter part 15B. That is, the first tubular part 41 can be inserted from the treatment tool insertion hole 18F into the first inner diameter part 15A, but cannot be inserted into the second inner diameter part 15B, so that the insertion into the forceps channel 15 is restricted.

The second tubular part 42 is provided at a base end part of the first tubular part 41, and is formed of a harder material than the first tubular part 41. The second tubular part 42 has a cylindrical shape having a larger outer diameter than the first tubular part 41. In the present embodiment, the first tubular part 41 and the second tubular part 42 are coupled to each other via the coupling member 43.

The coupling member 43 is formed in a cylindrical shape having an outer diameter larger than the second tubular part 42, the first tubular part 41 is firmly attached to a distal end side thereof, and the second tubular part 42 is firmly attached to a base end side thereof. A groove 43A (see Figs. 9A and 9B) is formed on an outer peripheral surface of the coupling member 43. As a result, the coupling member 43 is easily bent, and it is possible to prevent the coupling member 43 from interfering with the operation.

An inner peripheral surface of the second tubular part 42 is an inner peripheral side sliding part 42A that allows the treatment tool body 21 to move forward and backward with respect to the forceps channel 15. Specifically, the inner peripheral side sliding part 42A slides on the inner sheath 32 that is fitted to the treatment tool body 21. Therefore, the inner sheath 32 and the treatment tool body 21 slide integrally on the outer sheath 31. As described above, the treatment tool insertion hole 18F is the introduction port into the forceps channel 15 and slides on the outer peripheral side sliding part 41A of the first tubular part 41. Then, the inner sheath 32 slides on the first tubular part 41 that slides on the treatment tool insertion hole 18F, that is, the outer sheath 31. Therefore, the inner peripheral side sliding part 42A allows the treatment tool body 21 to move forward and backward with respect to the forceps channel 15 along with the inner sheath 32.

It should be noted that, in a case in which the treatment tool body 21 moves forward and backward, the inner peripheral side sliding part 42A has a function of actively moving the treatment tool body 21 by sliding (that is, a resistance force to the inner sheath 32 or the treatment tool body 21 is small), whereas the outer peripheral side sliding part 41A has a function of substantially not allowing the treatment tool body 21 to move forward and backward with respect to the forceps valve 18 (that is, a resistance force to the forceps valve 18 is large).

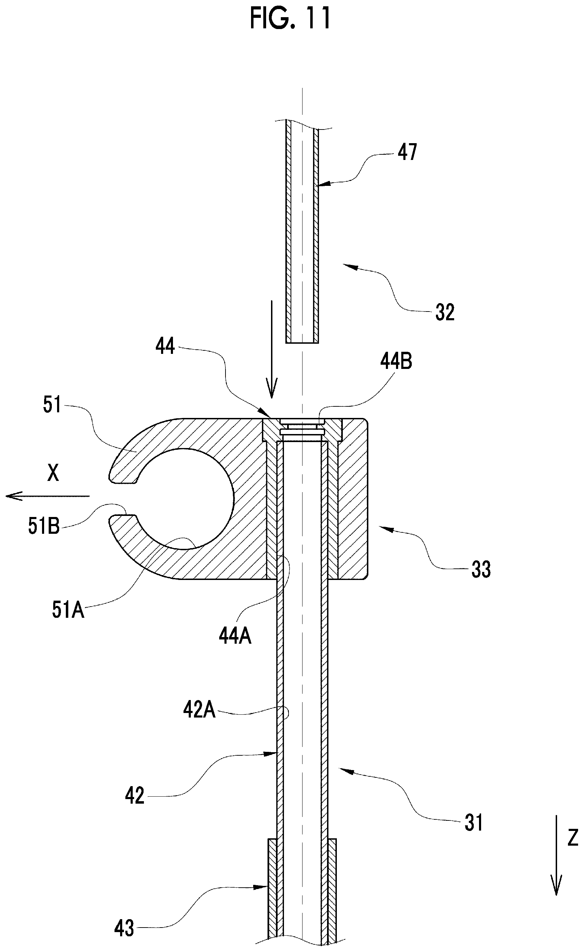

As shown in Fig. 11, the seal member 44 is provided at a base end part of the second tubular part 42. The seal member 44 is formed in a cylindrical shape, and the second tubular part 42 is firmly attached to a distal end side of an inner peripheral surface 44A. An annular protrusion 44B that protrudes radially inward is formed on a base end part of the inner peripheral surface 44A. An inner diameter of the protrusion 44B is slightly smaller than an outer diameter of a third tubular part 47, which will be described below, of the inner sheath 32.

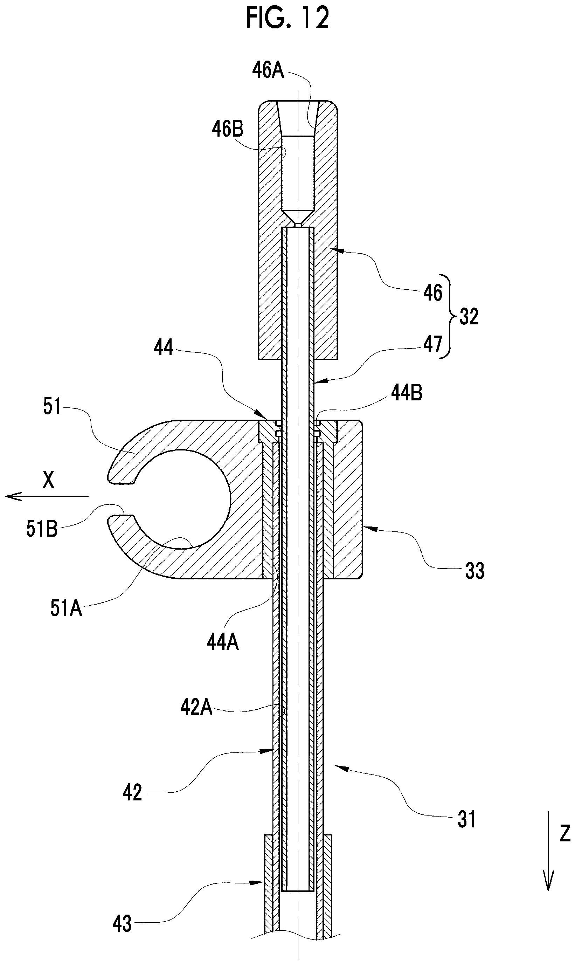

As shown in Fig. 12, in a case in which the third tubular part 47 of the inner sheath 32 is inserted into the inner peripheral side sliding part 42A of the outer sheath 31, the protrusion 44B is closely attached to the third tubular part 47, so that a gap between the outer sheath 31 and the inner sheath 32 is filled. As a result, during various treatments, the airtightness between the treatment tool body 21 and the oversheath 22 can be improved, and the air and carbon dioxide in the stomach can be prevented from escaping, the contents can be prevented from leaking, and a space for performing various treatments in a comfortable space for the doctor or the assistant can be secured, and contamination due to the leakage can be prevented. The attachment/detachment member 33 is fitted to an outer peripheral surface 44C of the seal member 44.

In the inner sheath 32, a fitting part 46 and the third tubular part 47 are provided integrally with each other. An inner peripheral surface of the fitting part 46 has a tapered part 46A and a circumferential part 46B. The tapered part 46A is located at a base end part of the fitting part 46. The tapered part 46A of the fitting part 46 is externally fitted to the tapered part 27A of the treatment tool body 21. The fitting part 46 is not limited to this, and need only be fitted to the operating part 26 of the treatment tool body 21 or the flexible sheath 23.

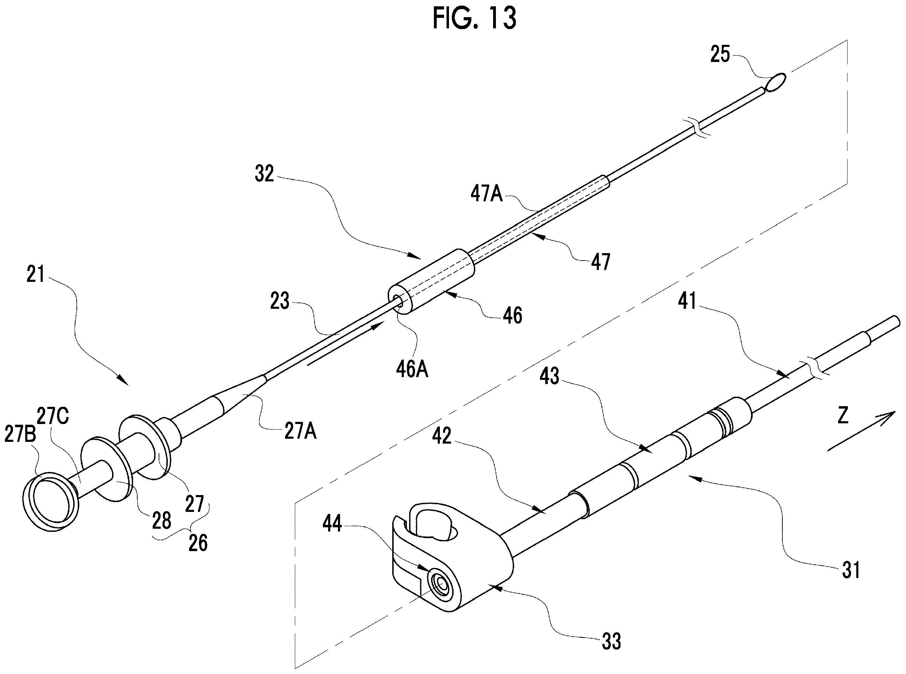

As shown in Fig. 13, the third tubular part 47 is formed in a circular tubular shape that covers at least a part of the flexible sheath 23. An outer peripheral surface 47A of the third tubular part 47 is formed to match an inner diameter of the inner peripheral side sliding part 42A (see Fig. 6). As a result, the third tubular part 47 slides on the inner peripheral side sliding part 42A.

As shown in Fig. 14, in the oversheath 22, the third tubular part 47 covers at least a part of the flexible sheath 23, and the fitting part 46 of the inner sheath 32 is externally fitted to the operating part 26 of the treatment tool body 21. The flexible sheath 23 is inserted into the first tubular part 41, and the third tubular part 47 is inserted into the inner peripheral side sliding part 42A (see Fig. 11). The treatment tool body 21 is movable forward and backward and rotatable with respect to the oversheath 22 within a range in which the third tubular part 47 and the inner peripheral side sliding part 42A slide. Further, in this state, the snare wire 25 can be brought into the expanded state or the contracted state by the push-pull operation of the operating wire 24 in response to the sliding movement of the slider 28.

It should be noted that, since an outer diameter of the fitting part 46 is larger than the outer diameter of the third tubular part 47, in a case in which the inner sheath 32 and the treatment tool body 21 slide on the outer sheath 31 toward the distal end side in the insertion direction Z, the fitting part 46 abuts on a base end of the outer sheath 31. As a result, the user can recognize that the inner sheath 32 and the treatment tool body 21 cannot further slide on the outer sheath 31 toward the distal end side in the insertion direction Z.

In addition, a length L13 of the third tubular part 47 in the insertion direction Z is longer than a range in which the treatment tool body 21 moves forward and backward with respect to the forceps channel 15. In addition, the length L13 of the third tubular part 47 is longer than a range in which the snare wire 25 protrudes from the distal end part 11A and is movable. In a case in which the range in which the treatment tool body 21 moves forward and backward with respect to the forceps channel 15 is set to 50 mm, the length L13 is preferably, for example, 75 mm. As a result, it is possible to sufficiently secure a range in which the third tubular part 47 and the inner peripheral side sliding part 42A slide, and it is possible to easily allow the treatment tool body 21 to move forward and backward and rotate with respect to the oversheath 22.

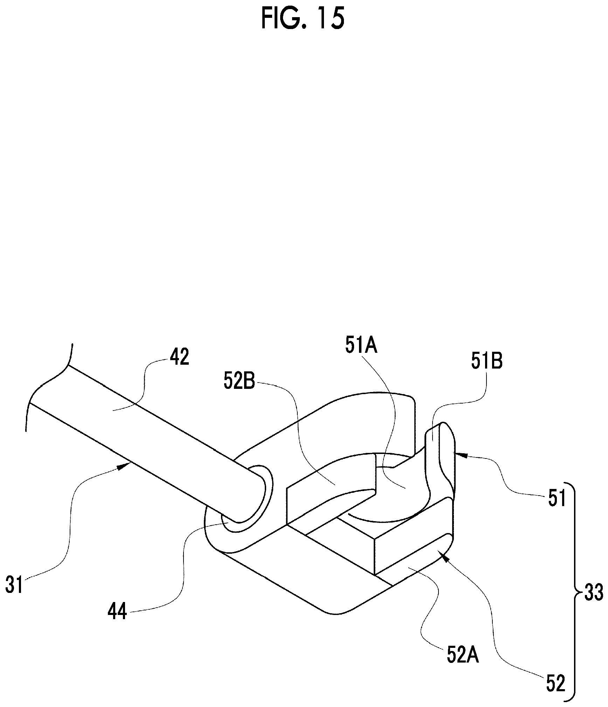

As shown in Fig. 15, the attachment/detachment member 33 is provided integrally with the outer sheath 31, and attachably and detachably engages with the forceps valve 18 mounted on the forceps channel 15. In a case in which the attachment/detachment member 33 engages with the forceps valve 18, the outer sheath 31 is attached to the endoscope 2 via the attachment/detachment member 33. Specifically, the attachment/detachment member 33 includes a first engaging part 51 that engages with the forceps valve 18, and a second engaging part 52 that engages with a part of the endoscope 2.

The first engaging part 51 is a C-shaped engaging part (see also Figs. 11 and 12). The first engaging part 51 includes, for example, an inner peripheral surface 51A, a notch 51B, and a recess part 51C. The inner peripheral surface 51A is formed to have an inner diameter matching an outer diameter of the forceps valve 18. The notch 51B is formed to be continuous with the inner peripheral surface 51A and to have a width smaller than the outer diameter of the forceps valve 18.

The first engaging part 51 is elastically deformed by being pushed in by aligning the notch 51B with the position of the forceps valve 18, thereby widening the width of the notch 51B. Further, in a case in which the first engaging part 51 is pushed in, the notch 51B passes over the forceps valve 18, and the inner peripheral surface 51A is fitted to the outer peripheral surface of the forceps valve 18. As a result, the first engaging part 51 engages with the forceps valve 18.

The recess part 51C is disposed at a position symmetrical to the notch 51B with the center of the inner peripheral surface 51A interposed therebetween. In a case in which the inner peripheral surface 51A is fitted to the outer peripheral surface of the forceps valve 18, the attachment arm 18C (see Fig. 3) is located in the notch 51B, and the grip part 18D (see Fig. 3) is located in the recess part 51C. The recess part 51C is a recess part that is continuous with the inner peripheral surface 51A and is recessed in a circular shape. The recess part 51C is disposed at a position corresponding to the grip part 18D of the forceps valve 18. As a result, in a case in which the outer peripheral surface of the forceps valve 18 is fitted to the inner peripheral surface 51A, the recess part 51C locks the grip part 18D. As a result, the attachment/detachment member 33 is restricted from rotating about the forceps valve 18. In this case, the notch 51B may lock the attachment arm 18C of the forceps valve 18.

As described above, the attachment/detachment member 33 is fitted to the seal member 44 of the outer sheath 31. An attachment direction X of the attachment/detachment member 33 with respect to the forceps valve 18 is a direction of the notch 51B as viewed from the center of the inner peripheral surface 51A. The attachment direction X (see Fig. 11) is orthogonal to the insertion direction Z, that is, the axial direction of the outer sheath 31.

The second engaging part 52 includes a pair of clamping pieces 52A and 52B that clamp a part of the endoscope 2. In the present embodiment, the pair of clamping pieces 52A and 52B are formed at an interval that matches the treatment tool introduction part 12A of the endoscope 2. In a case in which the second engaging part 52 pushes the attachment/detachment member 33 from the radial direction of the forceps valve 18, the pair of clamping pieces 52A and 52B clamp the treatment tool introduction part 12A. As a result, the second engaging part 52 engages with the treatment tool introduction part 12A. The attachment/detachment member 33 has a structure in which the first engaging part 51 and the second engaging part 52 overlap with each other.

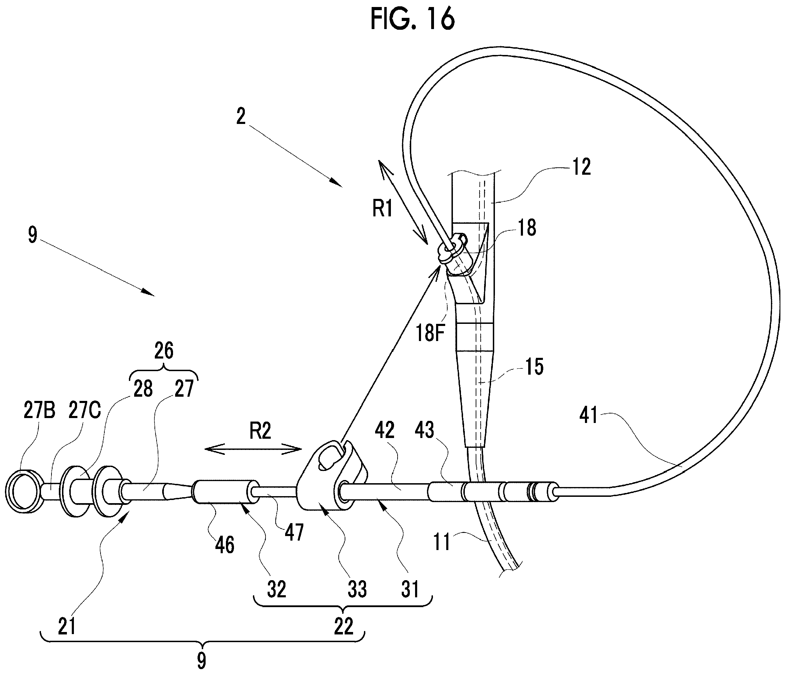

As shown in Fig. 16, the endoscopic treatment tool device 9 is inserted into the forceps channel 15 of the endoscope 2 in a state in which the inner sheath 32 and the treatment tool body 21 are inserted into the outer sheath 31. Specifically, the first tubular part 41 of the outer sheath 31 is inserted into the forceps channel 15 through the treatment tool insertion hole 18F or the like formed in the forceps valve 18.

By pushing the attachment/detachment member 33 from the radial direction of the forceps valve 18, the forceps valve 18 is fitted to the inner peripheral surface 51A of the first engaging part 51, and the treatment tool introduction part 12A is clamped by the clamping pieces 52A and 52B of the second engaging part 52. As a result, the attachment/detachment member 33 can engage with the forceps valve 18 and the treatment tool introduction part 12A. That is, it is possible to attach the outer sheath 31 to the endoscope 2 via the attachment/detachment member 33.

In the endoscope system 1, in a case in which the first resistance force in a case of allowing the first tubular part 41 to move forward and backward with respect to the forceps channel 15 is denoted by R1, and the second resistance force in a case of moving the inner sheath 32 and the treatment tool body 21 forward and backward with respect to the second tubular part 42 is denoted by R2, the first resistance force R1 is larger than the second resistance force R2. It should be noted that the first resistance force R1 referred to herein is mainly a frictional force generated between the forceps valve 18 and the outer sheath 31, and the second resistance force R2 is mainly a frictional force generated between the outer sheath 31 and the inner sheath 32. In order to make the first resistance force R1 larger than the second resistance force R2, for example, a material having a smooth surface (with low friction) need only be selected as the seal member 44 of the outer sheath 31, or a dimensional difference between the hole diameter of the forceps port and the outer diameter of the first tubular part 41 need only be adjusted. In addition, as a specific example that causes the difference between the first resistance force R1 and the second resistance force R2, in addition to the above-described case, for example, the first resistance force R1 can be made larger than the second resistance force R2 by making the outer diameter of the outer peripheral side sliding part 41A of the outer sheath 31 larger than the minimum diameter of the forceps channel 15, and making the outer diameter of the third tubular part 47 of the inner sheath 32 smaller than the inner diameter of the inner peripheral side sliding part 42A of the outer sheath 31. Alternatively, for example, the first resistance force R1 can be made larger than the second resistance force R2 by making a clearance between the inner peripheral surface 16B of the socket 16 and the outer diameter of the outer peripheral side sliding part 41A of the outer sheath 31 smaller than a clearance between the inner diameter of the inner peripheral side sliding part 42A of the outer sheath 31 and the third tubular part 47 of the inner sheath 32.

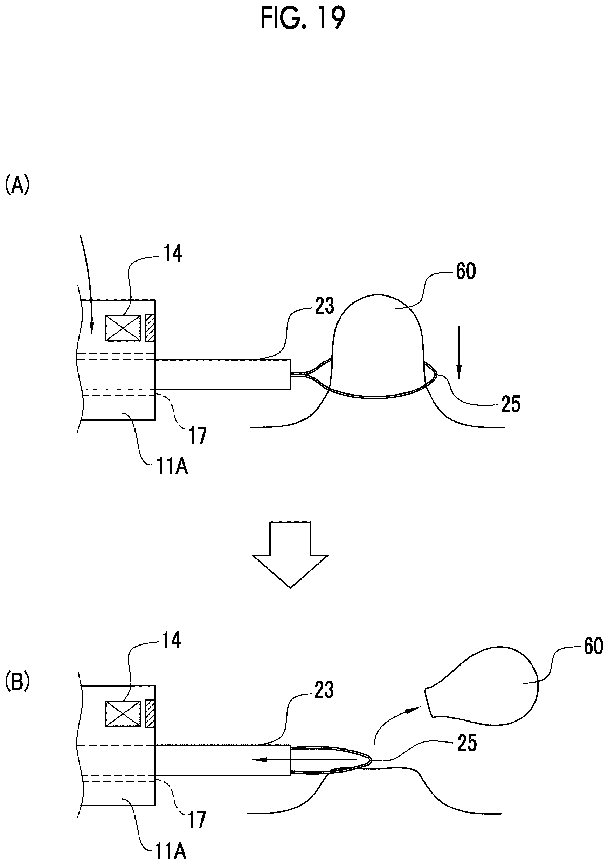

An operation using the endoscope system 1 in a case in which the doctor D, who is the operator, performs the treatment using the endoscopic treatment tool device 9 will be described. First, the doctor D inserts the insertion part 11 of the endoscope 2 into the body of the patient P, observes an endoscopic image captured by the image sensor 14, finds a region of interest, such as a lesion portion 60 (see Figs. 18 and 19), and determines a part to be treated. Then, as shown in Fig. 17, first, while maintaining a state in which the flexible sheath 23 of the treatment tool body 21 and the inner sheath 32 are inserted into the outer sheath 31, the doctor D inserts the outer sheath 31 and the flexible sheath 23 into the forceps channel 15. In this case, the doctor D brings the snare wire 25 into the contracted state by the operation of the operating part 26. Then, the doctor D attaches the outer sheath 31 to the operating part 12 of the endoscope 2 by engaging the attachment/detachment member 33 with the forceps valve 18.

As described above, the treatment tool body 21 can move forward and backward and rotate with respect to the outer sheath 31 within a range in which the third tubular part 47 and the inner peripheral side sliding part 42A slide. That is, in a case in which the oversheath 22 and the treatment tool body 21 are inserted into the forceps channel 15, the treatment tool body 21 can move forward and backward and rotate within a certain range with respect to the forceps channel 15. As a result, the doctor D can allow the treatment tool body 21 to move forward and backward with respect to the outer sheath 31 while observing the endoscopic image captured by the image sensor 14, and can align the position of the distal end part of the flexible sheath 23 with the position of the lesion portion 60 (state shown in (A) of Fig. 18). In a case of performing this operation, the doctor D can perform the operation by gripping the operating part 12 of the endoscope 2 with one hand DH1 and gripping the treatment tool body 21 with the other hand DH2.

Next, the operator operates the operating part 26 with the other hand DH2 to protrude the snare wire 25 or rotates the treatment tool body 21 with respect to the outer sheath 31 to align the position of the snare wire 25 with the position of the lesion portion 60 (state shown in (B) of Fig. 18). Further, the doctor D operates the endoscope 2 with the one hand DH1, for example, to bend the bendable part 11B. As a result, the position of the snare wire 25 is brought close to the position of the lesion portion 60, and the lesion portion 60 is surrounded by a loop of the snare wire 25 (state shown in (A) of Fig. 19). Then, the doctor D can operate the snare wire 25 to pull the snare wire 25 into the flexible sheath 23 to strangulate the lesion portion 60, and then operate the high-frequency power supply 8 to allow the high-frequency current to flow through the snare wire 25, thereby resecting the lesion portion 60 from the body of the patient P (state shown in (B) of Fig. 19).

As described above, in a case in which the treatment is performed by using the oversheath 22 and the endoscopic treatment tool device 9 including the oversheath 22, the doctor D can easily perform a plurality of types of treatments, that is, the operation of allowing the treatment tool body 21 to move forward and backward and rotate with respect to the forceps channel 15, and further the contraction and the expansion of the snare wire 25, in addition to the operation of the endoscope 2. The doctor D can perform the above-described treatment alone. As a result, it is possible to perform the treatment tool operation without the need for the cooperation, and in a case in which the doctor is the main operator, the treatment can be performed with a small number of people.

In addition, since the oversheath 22 comprises the first tubular part 41 that slides on the inner peripheral surface of the forceps port and the second tubular part 42 that allows the treatment tool body 21 to move forward and backward with respect to the forceps channel 15, it is not necessary to provide a dedicated component in the treatment tool body 21, and the treatment can be easily performed by combining the oversheath 22 with the treatment tool body 21. That is, the oversheath 22 can correspond to a plurality of types of endoscopic treatment tools.

In addition, since the oversheath 22 comprises the outer sheath 31 in which the first tubular part 41 and the second tubular part 42 are provided integrally with each other, and the inner sheath 32 that is externally fitted to the treatment tool body 21, slides on the second tubular part 42, and covers at least a part of the flexible sheath 23, the oversheath 22 can correspond to a plurality of types of endoscopic treatment tools, and the treatment tool operation can be easily performed.

Since the oversheath 22 can be attached to the operating part 12 via the attachment/detachment member 33 that engages with the forceps valve 18, the operation performed by the doctor D is concentrated in the vicinity of the operating part 12, and the operation of the treatment tool body 21 can be stably performed. In addition, since the attachment/detachment member 33 has the first engaging part 51 that engages with the forceps valve 18, and the second engaging part 52 that engages with a part of the endoscope 2, the oversheath 22 can be further reliably attached to the operating part 12.

Further, since the first resistance force R1 in a case of allowing the oversheath 22 to move forward and backward with respect to the forceps valve 18 is larger than the second resistance force R2 in a case of allowing the treatment tool body 21 to move forward and backward with respect to the oversheath 22, the movement of the oversheath 22 can be restricted in a case in which the treatment tool body 21 is operated. As a result, the operation of the treatment tool body 21 can be performed more stably.

### [Second embodiment]

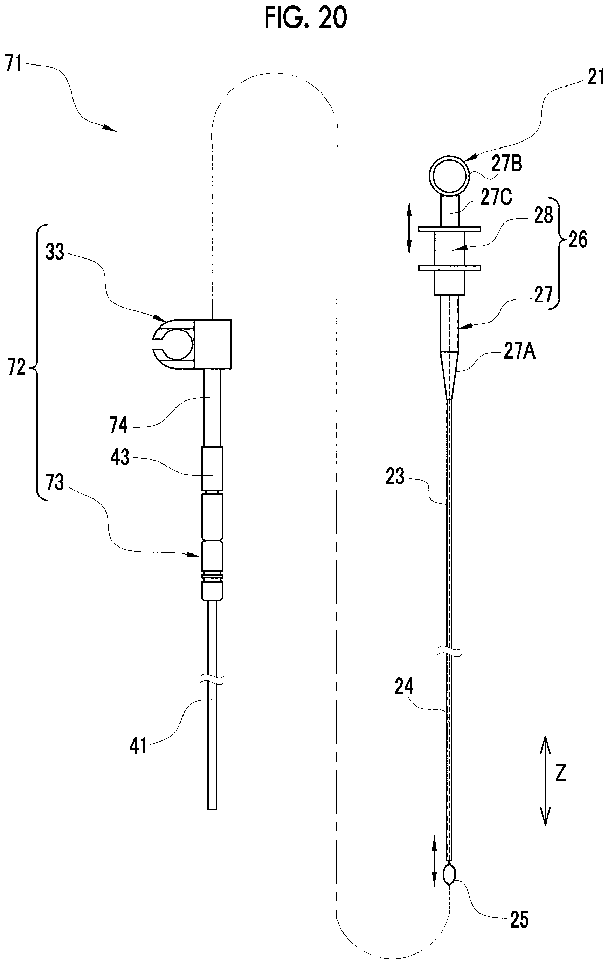

In the first embodiment, the oversheath 22 is externally fitted to the treatment tool body 21, and the inner sheath 32 that covers at least a part of the flexible sheath 23 is caused to slide on the outer sheath 31, thereby enabling the treatment tool operation. However, the present invention is not limited to this, and a configuration may be adopted in which the inner sheath is omitted, as in an endoscopic treatment tool device 71 shown in Fig. 20.

The endoscopic treatment tool device 71 comprises the treatment tool body 21 and an oversheath 72. The oversheath 72 comprises an outer sheath 73 and the attachment/detachment member 33. It should be noted that the configurations of the treatment tool body 21 and the attachment/detachment member 33 are the same as the configurations in the first embodiment, and the description thereof will be omitted. In addition, the same components of the outer sheath 73 as the components of the outer sheath 31 in the first embodiment are denoted by the same reference numerals, and the description thereof will be omitted.

In the outer sheath 73, the first tubular part 41, a second tubular part 74, the coupling member 43, and a seal member 75 are provided integrally with each other. The first tubular part 41, the second tubular part 74, the coupling member 43, and the seal member 75 are formed of, for example, a soft material, such as a soft resin.

The second tubular part 74 is provided at the base end part of the first tubular part 41, and is formed of a harder material than the first tubular part 41. In the present embodiment, the first tubular part 41 and the second tubular part 74 are coupled to each other via the coupling member 43.

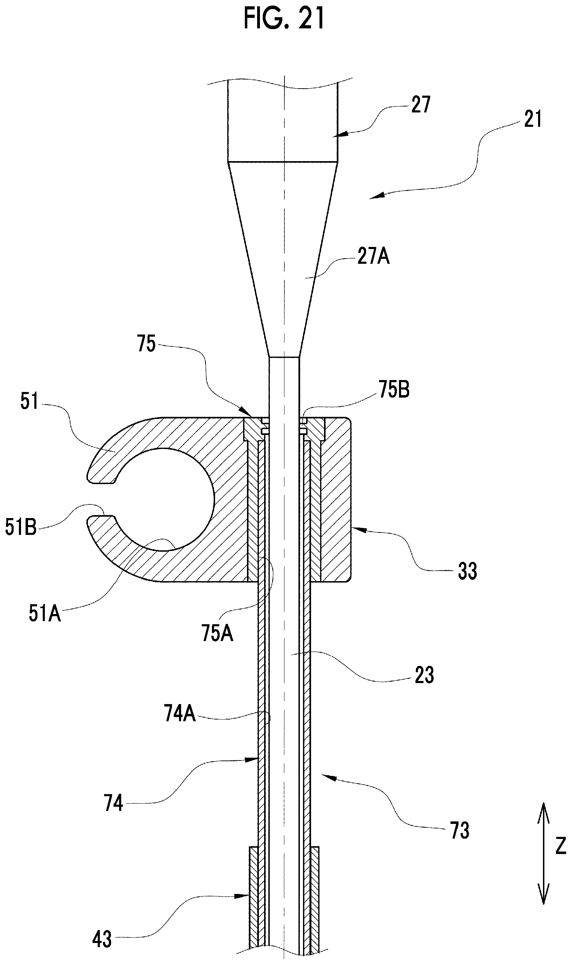

As shown in Fig. 21, an inner peripheral surface of the second tubular part 74 is an inner peripheral side sliding part 74A that allows the treatment tool body 21 to move forward and backward with respect to the forceps channel 15. Specifically, the inner peripheral side sliding part 42A slides on the flexible sheath 23 of the treatment tool body 21. As described above, the first tubular part 41 slides on the treatment tool insertion hole 18F, which is the introduction port into the forceps channel 15. Therefore, the inner peripheral side sliding part 74A allows the treatment tool body 21 to move forward and backward with respect to the forceps channel 15. It should be noted that the outer shape, the material, and the like of the second tubular part 74 are the same as the outer shape, the material, and the like of the second tubular part 42 of the outer sheath 31 in the first embodiment.

The seal member 75 is provided at a base end part of the second tubular part 74. The seal member 75 is formed in a cylindrical shape, and the second tubular part 74 is firmly attached to a distal end side of an inner peripheral surface 75A. An annular protrusion 75B that protrudes radially inward is formed on a base end part of the inner peripheral surface 75A. An inner diameter of the protrusion 75B is slightly smaller than an outer diameter of the flexible sheath 23.

In a case in which the flexible sheath 23 is inserted into the inner peripheral side sliding part 74A, the protrusion 75B is closely attached to the flexible sheath 23, so that a gap between the outer sheath 73 and the flexible sheath 23 is filled. As a result, during various treatments, the airtightness between the treatment tool body 21 and the oversheath 72 can be improved, and the air and carbon dioxide in the stomach can be prevented from escaping, the contents can be prevented from leaking, and a space for performing various treatments in a comfortable space for the doctor or the assistant can be secured, and contamination due to the leakage can be prevented. The attachment/detachment member 33 is fitted to an outer peripheral surface 75C of the seal member 75.

In a case of using the endoscopic treatment tool device 71 including the oversheath 22, the first resistance force in a case of allowing the first tubular part 41 to move forward and backward with respect to the forceps channel 15 is larger than the second resistance force in a case of allowing the treatment tool body 21 to move forward and backward with respect to the second tubular part 74. It should be noted that the first resistance force is mainly a frictional force generated between the forceps valve 18 and the outer sheath 73, and the second resistance force is mainly a frictional force generated between the outer sheath 73 and the flexible sheath 23. In addition, the attachment/detachment member 33 can engage with the forceps valve 18 and the treatment tool introduction part 12A as in the first embodiment. That is, the outer sheath 73 can be attached to the endoscope 2 via the attachment/detachment member 33.

With the above-described configuration, the treatment tool body 21 can move forward and backward and rotate with respect to the outer sheath 73 within a range in which the flexible sheath 23 and the inner peripheral side sliding part 74A slide. That is, in a case in which the oversheath 72 and the treatment tool body 21 are inserted into the forceps channel 15, the treatment tool body 21 can move forward and backward and rotate within a certain range with respect to the forceps channel 15. Therefore, the doctor who is the user can easily perform a plurality of types of treatments, such as the operation of allowing the treatment tool body 21 to move forward and backward and rotate with respect to the forceps channel 15, and further the contraction and expansion of the snare wire 25, in addition to the operation of the endoscope 2, as in the first embodiment.

### [First modification example]

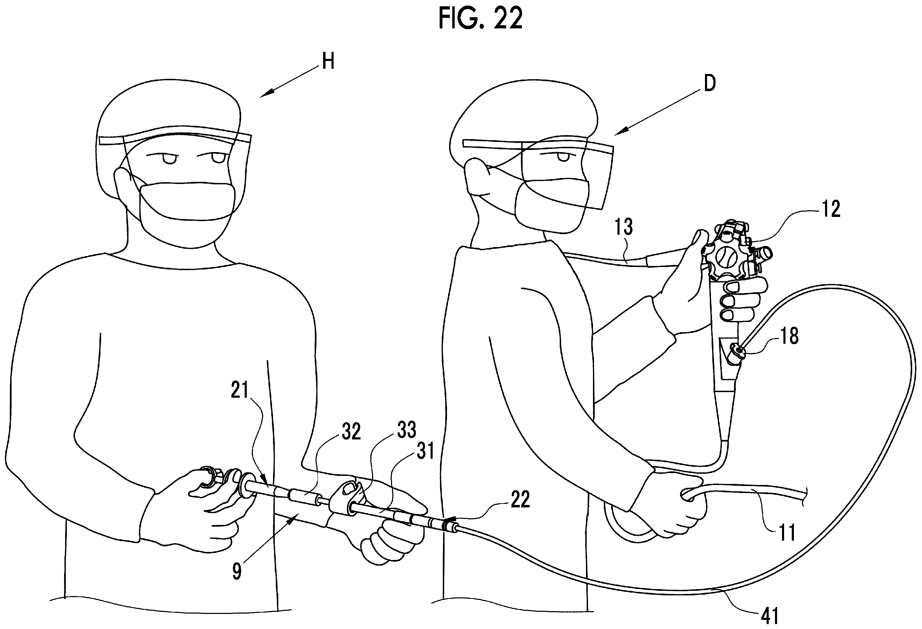

In the first and second embodiments, the example has been described in which the doctor D performs a plurality of types of treatments alone, but the present invention is not limited to this, and the treatment may be performed by the doctor D and an assistant H. For example, as shown in Fig. 22, in a case in which the doctor D operates the endoscope 2 with both hands, the assistant H can operate the endoscopic treatment tool device 9. That is, with the configuration of the endoscopic treatment tool device 9 according to the first and second embodiments, the assistant H can perform a plurality of types of treatments, such as an operation of sliding and rotating the treatment tool body 21 with respect to the forceps channel 15, the contraction and the expansion of the snare wire 25, and further the power supply operation of the snare wire 25 via the high-frequency power supply 8. That is, the number of the types of treatment tool operations that can be performed by the assistant is increased, and the treatment tool operation can be performed without the need for the cooperation. As described above, it is possible to cope with not only a case in which the doctor D performs the treatment alone but also a case in which the treatment is performed by a plurality of people. In this case, it is preferable that the assistant H removes the oversheath 22 from the operating part 12 of the endoscope 2 by releasing the engagement between the forceps valve 18 and the attachment/detachment member 33, so that the assistant H can freely handle the oversheath 22 and the treatment tool body 21. In addition, in the example shown in Fig. 22, a position of the doctor D and a position of the assistant H are close to each other, but the position of the doctor D and the position of the assistant H may be separated from each other.

### [Second modification example]

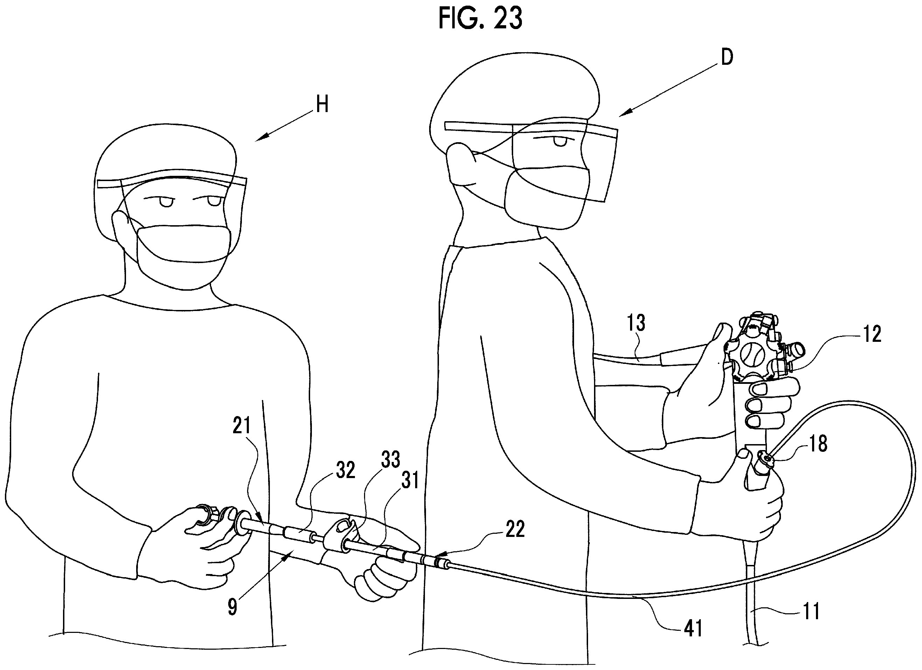

As shown in Fig. 23, the doctor D and the assistant H may cooperate with each other to perform the treatment. For example, the doctor D can perform the operation of the endoscope 2, the insertion of the oversheath 22 into the forceps channel 15, the power supply operation of the snare wire 25 via the high-frequency power supply 8, and the like, and the assistant H can perform the operation of allowing the treatment tool body 21 to move forward and rotate with respect to the forceps channel 15, and further the contraction and expansion of the snare wire 25, so that the operations related to the endoscopic treatment tool device 9 can be shared. In this case, it is preferable that the assistant H removes the oversheath 22 from the operating part 12 of the endoscope 2 by releasing the engagement between the forceps valve 18 and the attachment/detachment member 33, so that the assistant H can freely handle the oversheath 22 and the treatment tool body 21. In addition, in the example shown in Fig. 23, the position of the doctor D and the position of the assistant H are close to each other, but the position of the doctor D and the position of the assistant H may be separated from each other.

### [Third modification example]

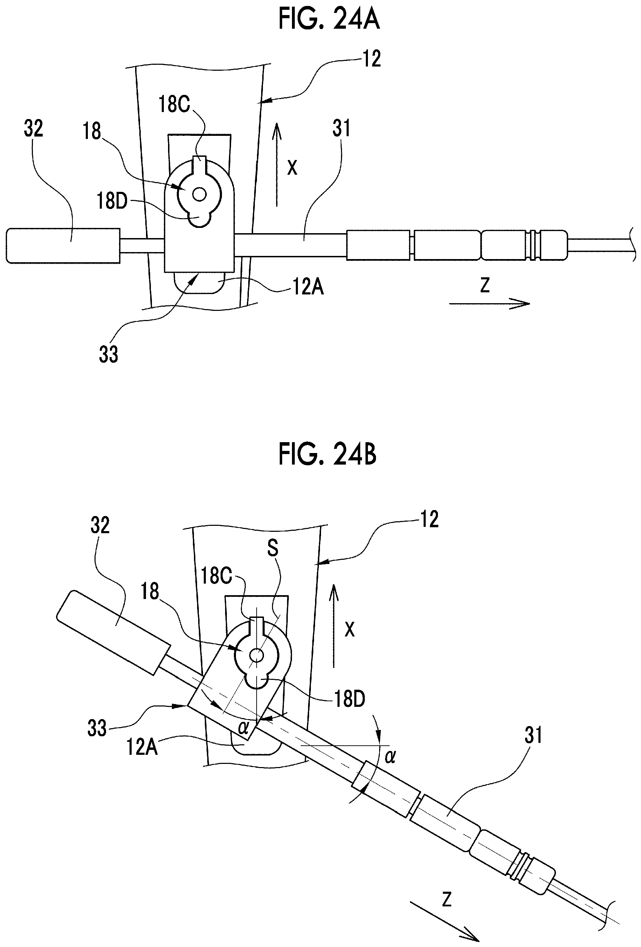

In each of the above-described embodiments, the attachment/detachment member 33 is disposed in parallel to the attachment direction X to the forceps valve 18. The attachment direction X is orthogonal to the insertion direction Z, that is, the axial direction of the outer sheath 31. As a result, as shown in Fig. 24A, in a case in which the outer sheath 31 is attached to the operating part 12 via the attachment/detachment member 33, the disposition is that the axial direction (insertion direction Z) of the outer sheath 31 is orthogonal to a direction (attachment direction X) from the forceps valve 18 toward the center of the operating part 12.

On the other hand, as shown in Fig. 24B, the present invention is not limited to this, and a configuration may adopted in which the attachment/detachment member 33 is inclined with respect to the attachment direction X to the forceps valve 18 (direction from the grip part 18D toward the attachment arm 18C in a case in which the attachment/detachment member 33 is attached to the forceps valve 18). Specifically, a surface S (surface orthogonal to the insertion direction Z) passing through the center of the attachment/detachment member 33 is inclined at an inclined angle α with respect to the attachment direction X. In addition, it can also be said that the attachment/detachment member 33 is disposed at a position at which the axial direction (insertion direction Z) of the outer sheath 31 is inclined at an inclined angle α from the direction orthogonal to the attachment direction X. In addition, in this case, for example, one of a plurality of the attachment/detachment members 33 in which the inclined angle α with respect to the attachment direction X is changed may be selected such that the inclined angle α can be changed in accordance with the preference of the user. The inclined angle α is preferably, for example, about 30° or 45°.

### [Fourth modification example]

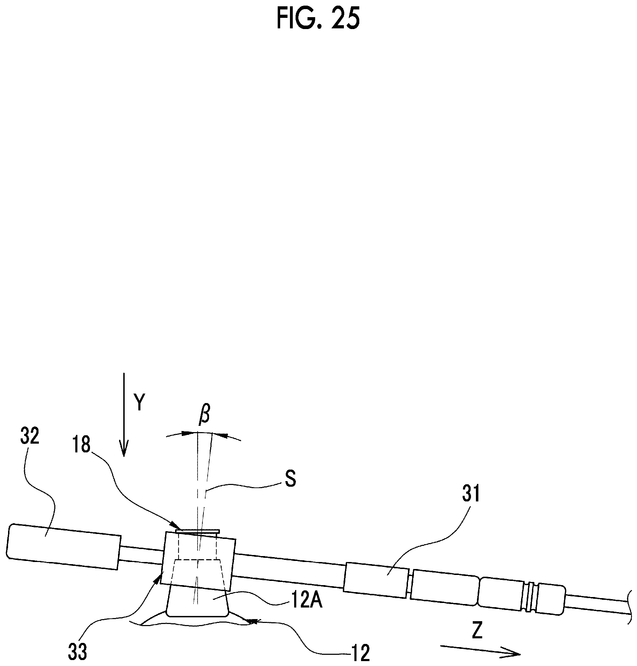

In addition, as shown in Fig. 25, a configuration may be adopted in which the attachment/detachment member 33 is inclined with respect to the axial direction Y of the forceps valve 18. Specifically, a surface S (surface orthogonal to the insertion direction Z) passing through the center of the attachment/detachment member 33 is inclined at an inclined angle β with respect to the axial direction Y of the forceps valve 18.

### [Fifth modification example]

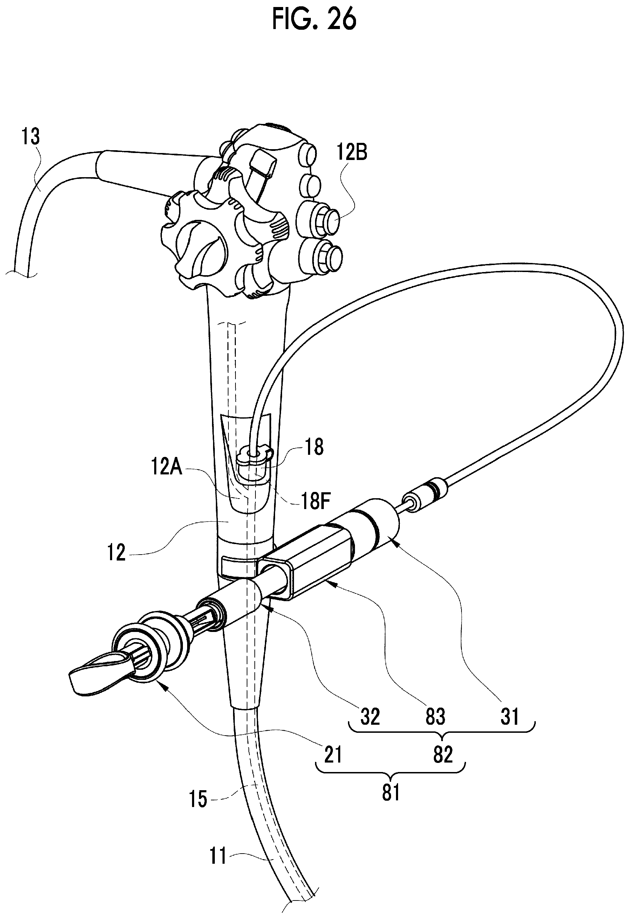

In the first and second embodiments, the example has been described in which the oversheath is attached to the endoscope 2 by engaging the attachment/detachment member 33 with the forceps valve 18, but the present invention is not limited to this. A configuration may be adopted in which the oversheath is attached by engaging the attachment/detachment member with the operating part, for example, a configuration may be adopted in which an attachment/detachment member 83 that engages with the operating part 12 is provided as shown in Fig. 26. It should be noted that the configurations of the oversheath 82 excluding the attachment/detachment member 83 and the treatment tool body 21 are the same as the configurations in the first embodiment, the same components, members, and the like are denoted by the same reference numerals, and thus the description thereof will be omitted.

An endoscopic treatment tool device 81 comprises the treatment tool body 21 and the oversheath 82. The oversheath 82 comprises the outer sheath 31, the inner sheath 32, and the attachment/detachment member 83. The outer sheath 31 and the inner sheath 32 have the same configuration as the oversheath 22 according to the first embodiment. It should be noted that the present invention is not limited to this, and a combination of the configuration in which the outer sheath 73 and the like, which are the same as the oversheath 72 in the second embodiment, are provided and the attachment/detachment member 83 may be adopted as the oversheath 82.

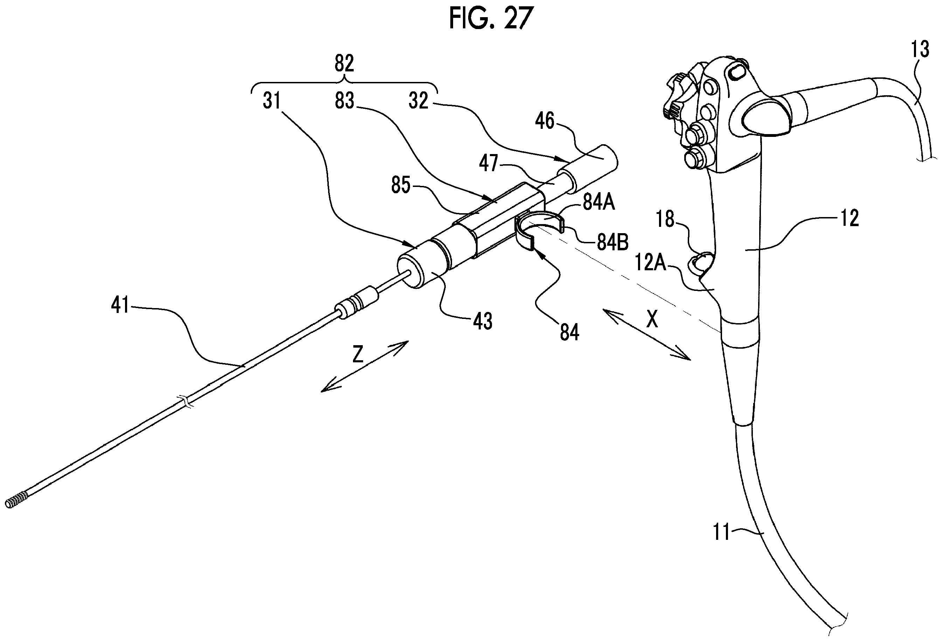

As shown in Fig. 27, the attachment/detachment member 83 is provided integrally with the outer sheath 31, and attachably and detachably engages with the operating part 12. In a case in which the attachment/detachment member 83 engages with the operating part 12, the outer sheath 31 is attached to the endoscope 2 via the attachment/detachment member 83. The attachment/detachment member 83 includes an engaging part 84 and a fitting part 85. The engaging part 84 engages with the operating part 12. Specifically, the engaging part 84 engages with a cylindrical outer peripheral surface of the operating part 12, excluding an operation dial, a button, and the treatment tool introduction part 12A.

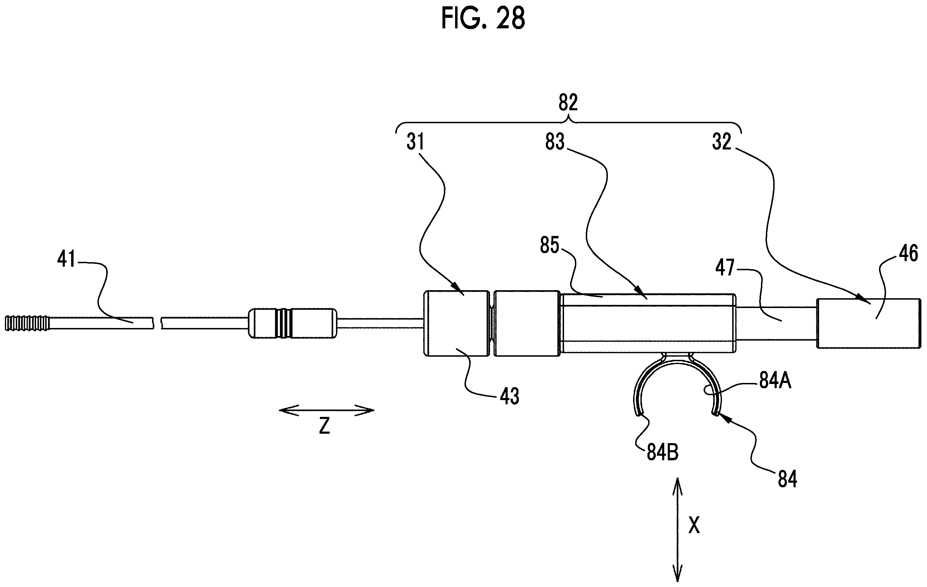

As shown in Fig. 28, the engaging part 84 is a C-shaped engaging part. The engaging part 84 has, for example, an inner peripheral surface 84A and a notch 84B. The inner peripheral surface 84A is formed with an inner diameter matching the outer diameter of the operating part 12. The notch 84B is formed to be continuous with the inner peripheral surface 84A and to have a width smaller than the outer diameter of the operating part 12. It should be noted that the fitting part 85 is fitted to the seal member 44 (see Fig. 6) of the outer sheath 31, which is not shown. For example, the attachment direction X of the attachment/detachment member 83 with respect to the operating part 12 is a direction of the notch 84B as viewed from the center of the inner peripheral surface 84A. This attachment direction X is orthogonal to the insertion direction Z, that is, the axial direction of the outer sheath 31.

The engaging part 84 is elastically deformed by being pushed in by aligning the notch 84B with the position of the operating part 12, thereby widening the width of the notch 84B. Further, in a case in which the engaging part 84 is pushed in, the notch 84B passes over the operating part 12, and the inner peripheral surface 84A is fitted to the outer peripheral surface of the operating part 12. As a result, the engaging part 84 engages with the operating part 12. That is, the oversheath 82 can be attached to the operating part 12 via the attachment/detachment member 83. Therefore, the operation performed by the doctor D is concentrated in the vicinity of the operating part 12, and the operation of the treatment tool body 21 can be stably performed. In addition, since the configurations of the outer sheath 31 and the inner sheath 32 constituting the oversheath 22 are the same as the configurations in the first embodiment, the same effects can be obtained in the treatment tool operation.

It should be noted that the present invention is not limited to the above-described configuration, and a combination of the configuration in which the outer sheath 73 and the like, which are the same as the oversheath 72 in the second embodiment, are provided and the attachment/detachment member 83 may be adopted as the oversheath 82. As a result, it is possible to obtain the same effects as the effects of the second embodiment through the treatment tool operation.

### [Third embodiment]

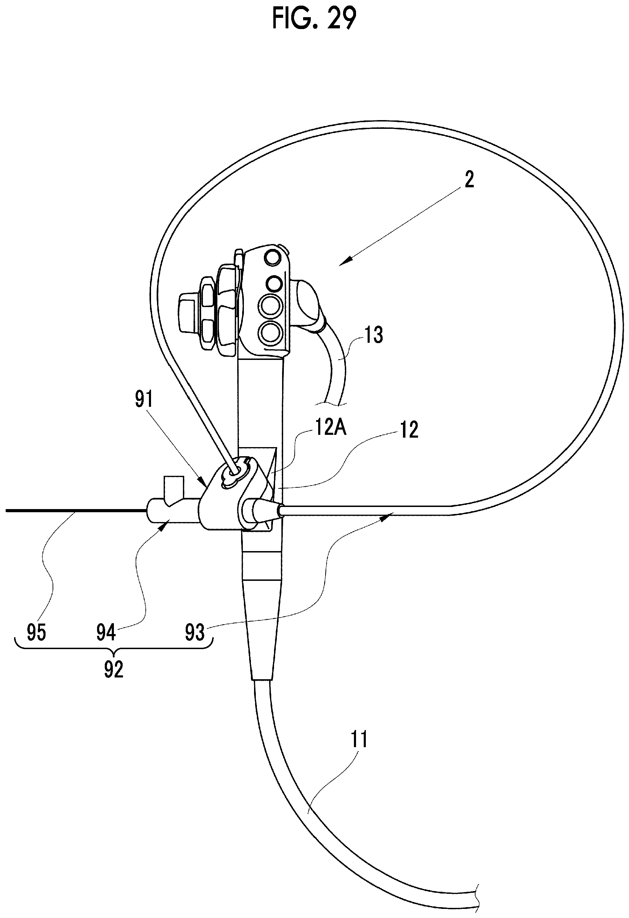

In the first and second embodiments, the configuration has been described in which the oversheath and the endoscopic treatment tool device including the oversheath are attached to the endoscope via the attachment/detachment member, but the present invention is not limited to this, and a configuration may be adopted in which an endoscopic instrument other than the oversheath is attached to the endoscope via the attachment/detachment member. In the present embodiment, as shown in Fig. 29, a configuration is adopted in which a catheter device 92 is attached via an attachment/detachment member 91. The catheter device 92 corresponds to an endoscopic instrument in the scope of the claims.

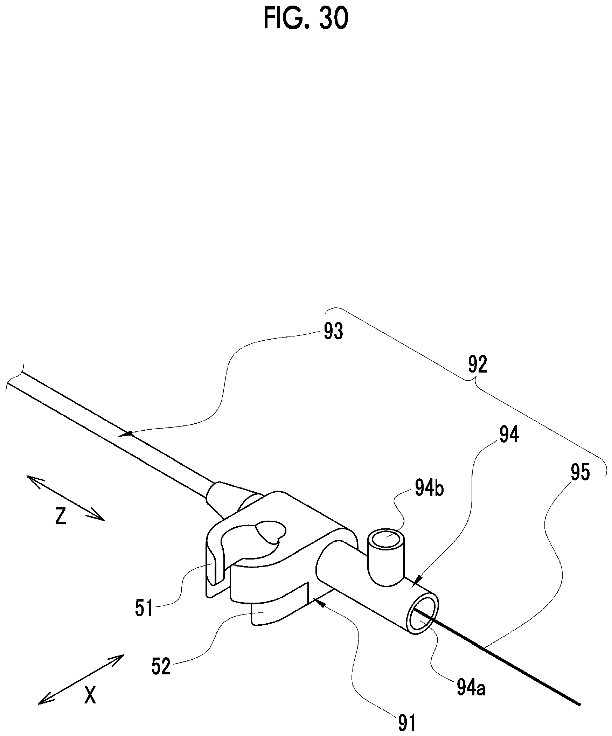

As shown in Fig. 30, the catheter device 92 comprises a guide catheter 93, a guide catheter cock 94, and a guide wire 95. The guide catheter 93 is formed of a flexible synthetic resin material, such as a fluorine-based resin or a nylon-based resin. A guide wire lumen is formed over the entire length of the guide catheter 93. The guide catheter cock 94 is provided integrally with each other at a base end of the guide catheter 93. The guide catheter cock 94 has a guide wire opening 94a and a liquid feeding socket 94b that communicate with the guide wire lumen.

The guide wire 95 is inserted into the guide wire lumen of the guide catheter 93 from the guide wire opening 94a. The guide catheter 93 is inserted into the forceps channel 15 of the endoscope 2 through the treatment tool insertion hole 18F. The guide wire 95 protrudes from the distal end part 11A of the endoscope 2 in a state of being inserted into the guide wire lumen of the guide catheter 93. In a state in which the insertion part 11 of the endoscope 2 is inserted into a body cavity of the patient, the doctor grips and operates the guide catheter 93 and the guide catheter cock 94. The guide catheter 93 is guided into the body cavity with the guide wire 95 as a guide. As a result, it is possible to perform liquid feeding into the body cavity by using the guide catheter 93.

The guide catheter cock 94 corresponds to a treatment tool operating part in the claims, and is an operating part that operates the catheter device 92. The attachment/detachment member 91 has the first engaging part 51 and the second engaging part 52, similarly to the attachment/detachment member 33 in the first and second embodiments. The first engaging part 51 engages with the forceps valve 18, and the second engaging part 52 engages with the treatment tool introduction part 12A. The treatment tool introduction part 12A is located on a proximal end side of the forceps valve 18. The attachment/detachment member 91 is provided integrally with the guide catheter cock 94, and attachably and detachably engages with the forceps valve 18. Specifically, the attachment/detachment member 91 is fitted to the outer peripheral surface of the guide catheter cock 94. As a result, the guide catheter cock 94 is disposed at a position at which the axial direction (insertion direction Z) of the guide catheter cock 94 intersects with the attachment/detachment direction (attachment direction X) of the attachment/detachment member 83 (see Fig. 30).

In a case in which the treatment is performed by using the catheter device 92, the doctor attaches the guide catheter cock 94 to the operating part 12 of the endoscope 2 by engaging the attachment/detachment member 91 with the forceps valve 18. As a result, the doctor can easily perform the treatment with the catheter device 92, such as the operation of guiding the guide catheter 93 into the body cavity, in addition to the operation of the endoscope 2.

Since the catheter device 92 can be attached to the operating part 12 via the attachment/detachment member 91, the operation performed by the doctor is concentrated in the vicinity of the operating part 12, and the operation of the catheter device 92 can be stably performed. Since the attachment/detachment member 91 has the first engaging part 51 and the second engaging part 52, the catheter device 92 can be reliably attached to the operating part 12. It should be noted that, in Fig. 29, the guide catheter cock 94 is disposed in a direction intersecting with the treatment tool introduction part 12A, but the present invention is not limited to this, and the guide catheter cock 94 may be disposed in a direction along the treatment tool introduction part 12A.

### [Fourth embodiment]

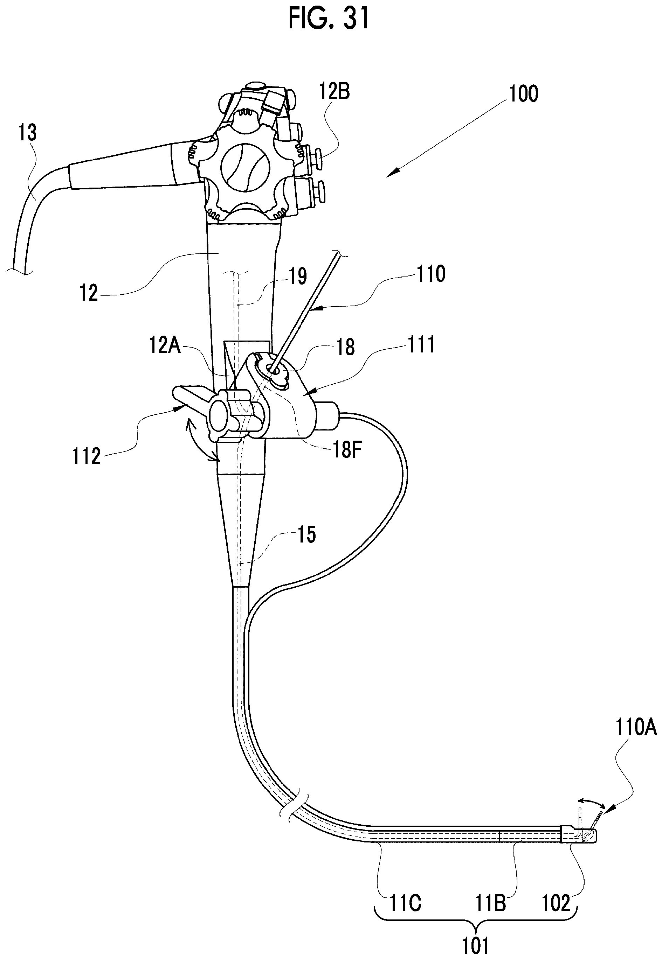

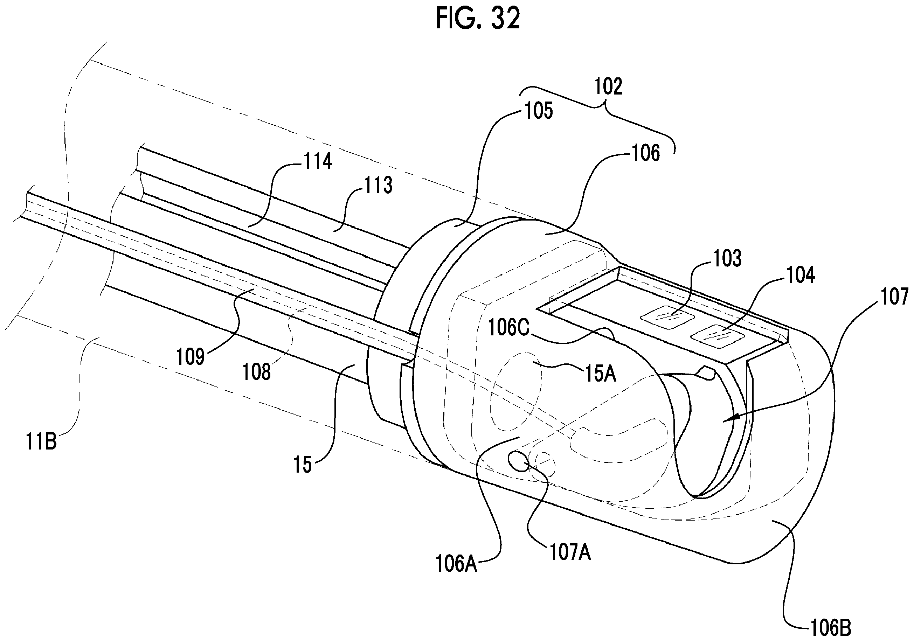

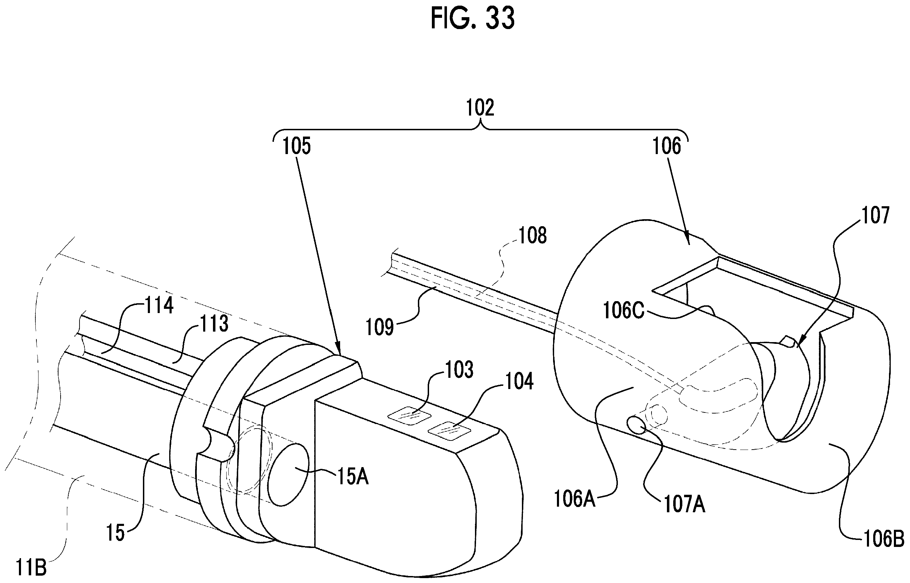

The endoscopic instrument to be attached to the endoscope via the attachment/detachment member is not limited to the example described in each of the above-described embodiments, and a configuration may be adopted in which an elevator wire operating part 112 is attached to an endoscope 100 via an attachment/detachment member 111 as shown in Fig. 31. The attachment/detachment member 111 has the same configuration as the attachment/detachment member 91 in the third embodiment. The elevator wire operating part 112 is connected to an elevator operating wire 108 (see Figs. 32 and 33) and operates an elevator 107, which will be described below.

The endoscope 100 comprises an insertion part 101, the operating part 12, and the universal cord 13. It should be noted that the operating part 12 and the universal cord 13 have the same configurations as the configurations of the endoscope 2 according to the first embodiment. The insertion part 101 consists of a distal end part 102, the bendable part 11B, and the flexible tube part 11C. It should be noted that the bendable part 11B and the flexible tube part 11C have the same configurations as the configurations of the insertion part 11 in the endoscope 2 according to the first embodiment.

As shown in Fig. 32, an image sensor 103, an illumination optical system 104, and the like are provided on the distal end part 102. The endoscope 100 is a side-viewing endoscope used as, for example, a duodenal endoscope, and the distal end part 102 shown in Figs. 32 and 33 has a configuration of the side-viewing endoscope. The forceps channel 15 is also disposed in the insertion part 101, as in the insertion part 11 in the endoscope 2 according to the first embodiment. The forceps channel 15 has one end connected to the distal end part 102, and the other end connected to the socket 16.

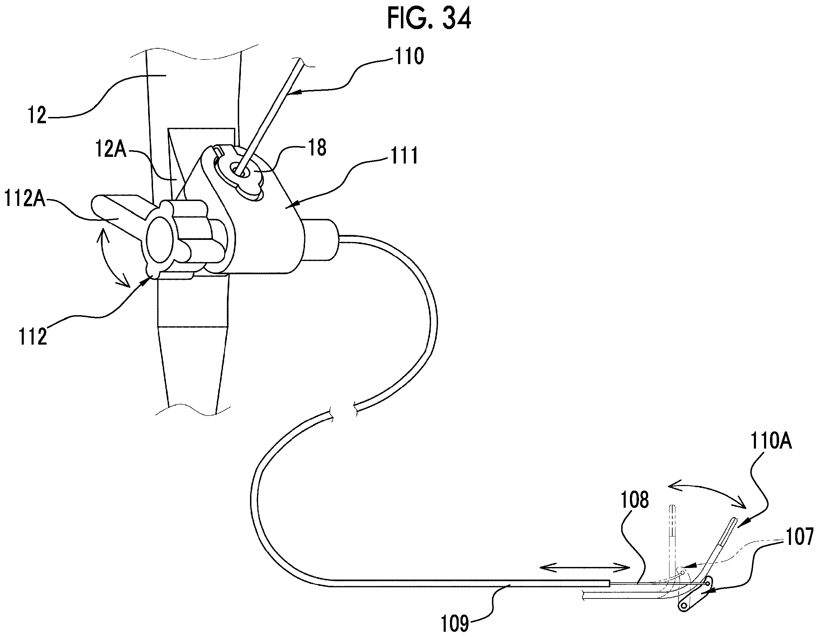

As shown in Fig. 33, the distal end part 102 includes a distal end part body 105 and a cap 106, and is configured by mounting the cap 106 on the distal end part body 105. The cap 106 is attachably and detachably mounted on the distal end part body 105. The distal end part body 105 is provided on a distal end side of the bendable part 11B. The cap 106 is provided with the elevator 107, the elevator operating wire 108, and a guide tube 109. The forceps channel 15 guides a distal end part 110A (see Figs. 31 and 34) of a treatment tool 110 into the inside of the distal end part 102. The treatment tool 110 is, for example, a contrast tube or a papillotomy knife.

The cap 106 is formed in a substantially cylindrical shape in which a distal end side is sealed, and has a peripheral surface part 106A and an end surface part 106B. A rectangular opening window 106C is formed in a part of the peripheral surface part 106A. In the example shown in Figs. 32 and 33, the opening window 106C is an opening portion cut out from the peripheral surface part 106A to the end surface part 106B.

The elevator 107 is attached to the inside of the cap 106 to be rotationally movable via a rotational movement shaft 107A and is movable between an elevated position and a reclined position. A distal end of the elevator operating wire 108 is coupled to an end part of the elevator 107. The elevator operating wire 108 is an operating wire for performing the operation of rotationally moving the elevator 107. The elevator operating wire 108 is inserted into the guide tube 109 and moves forward and backward inside the guide tube 109. The elevator operating wire 108 and the guide tube 109 are led out from the base end of the cap 106 to the outside, and the part led out to the outside from the cap 106 is disposed along the outer peripheral surface of the insertion part 101.

In a case in which the cap 106 is mounted on the distal end part body 105, a treatment tool outlet port 15A of the forceps channel 15 communicates with the inside of the cap 106 and the opening window 106C. The image sensor 103 and the illumination optical system 104 are exposed through the opening window 106C. The cap 106 is coaxially mounted on the distal end part body 105.

In addition to the image sensor 103 and the illumination optical system 104, a signal cable 113 and a light guide 114 are provided in the distal end part body 105. The signal cable 113 and the light guide 114 are each connected to the processor device 3 and the light source device 4 through the insertion part 101, the operating part 12, the connector (not shown), and the like. The processor device 3 performs image processing or the like on the imaging signal acquired by the image sensor 103 to display an observation image on the display 5. The light guide 114 is composed of an optical fiber cable or the like, transmits illumination light emitted from the light source device 4, and transmits the illumination light to an observation target via the illumination optical system 104.

As shown in Fig. 34, the elevator wire operating part 112 performs an operation of pushing and pulling the elevator operating wire 108 in response to a rotational movement operation of an operation lever 112A. The elevator 107 rotationally moves by pushing and pulling the elevator operating wire 108. The elevator 107 elevates the distal end part 110A of the treatment tool 110 led out from the treatment tool outlet port 15A.

The elevator 107 rotates from the reclined position (position shown by a solid line) to the elevated position (position shown by a two-dot chain line) by the elevator operating wire 108 being operated in a push-pull manner. As a result, the lead-out direction of the distal end part 110A of the treatment tool 110, which is led out to the treatment tool outlet port 15A, can be changed.

The elevator wire operating part 112 corresponds to a treatment tool operating part in the claims, and is an operating part for operating the treatment tool 110. The attachment/detachment member 111 includes the first engaging part 51 and the second engaging part 52, similarly to the attachment/detachment member 33 in the first and second embodiments and the attachment/detachment member 91 in the third embodiment. The attachment/detachment member 111 is provided integrally with the elevator wire operating part 112, and attachably and detachably engages with the forceps valve 18. Specifically, the attachment/detachment member 111 is fitted to the outer peripheral surface of the elevator wire operating part 112.

In a case in which the treatment is performed by using the treatment tool 110 and the elevator wire operating part 112, the doctor engages the attachment/detachment member 111 to the forceps valve 18 to attach the elevator wire operating part 112 to the operating part 12 of the endoscope 100. As a result, the doctor can easily perform the treatment by using the treatment tool 110 and the elevator wire operating part 112, such as the operation of rotationally moving the elevator 107, in addition to the operation of the endoscope 100. In addition, since the attachment/detachment member 111 has the same configuration as the attachment/detachment member 91 in the third embodiment, the same effects as the effects in the third embodiment can be obtained in the endoscope 100 and the elevator wire operating part 112 according to the present embodiment.

In each of the above-described embodiments, the treatment tool insertion hole 18F has been described as the forceps port, but the present invention is not limited to this, and the forceps port may be, for example, the inner peripheral surface 16B of the socket 16 connected to the forceps channel 15 (see Fig. 3).

In each of the above-described embodiments, the high-frequency snare that comprises the snare wire 25 as the treatment part and expands in a loop shape in a case of protruding from the distal end of the flexible sheath 23 by the push-pull operation of the operating wire 24 has been described as the treatment tool body 21, but the treatment tool body 21 is not limited to this, and may be, for example, a puncture needle, a treatment tool comprising an ultrasound oscillator, and the like. Further, it is preferable that a treatment part that is operated by the push-pull operation of the operating wire 24 is provided, and for example, a biopsy forceps for clamping and resecting a biological tissue in a body, a high-frequency scissors forceps that applies a high-frequency current to the treatment part, a high-frequency needle-like knife, or the like may be applied as the treatment tool body 21. In addition, the endoscopic instrument attached to the endoscope via the attachment/detachment member is not limited to the endoscopic instrument according to the third and fourth embodiments, and for example, a biopsy forceps, a high-frequency snare, a puncture needle, a treatment tool comprising an ultrasound oscillator, a high-frequency scissors forceps, a high-frequency needle knife, or the like may be applied. In addition, in each of the above-described embodiments, the upper gastrointestinal tract endoscope has been described as an example of the endoscope, but the present invention is not limited to this, and any endoscope comprising the forceps valve may be used, such as a bronchoscope or a lower gastrointestinal endoscope.

### Explanation of References

1: endoscope system
2: endoscope
3: processor device
4: light source device
5: display
6: user interface (UI)
7: suction device
8: high-frequency power supply
9: endoscopic treatment tool device
11: insertion part
11A: distal end part
11B: bendable part
11C: flexible tube part
12: operating part
12A: treatment tool introduction part
12B: operation button
13: universal cord
13A: connector
14: image sensor
15: forceps channel
15A: first inner diameter part
15B: first inner diameter part
15B: second inner diameter part
15C: branch part
16: socket
16A: flange part
16B: inner peripheral surface
17: forceps outlet
18: forceps valve
18A: valve body
18B: cap
18C: attachment arm
18D: grip part
18E: slit valve
18F: treatment tool insertion hole
19: suction channel
21: treatment tool body
22: oversheath
23: flexible sheath
24: operating wire
25: snare wire
26: operating part
27: operating part body
27A: tapered part
27B: finger hook part
27C: cylindrical part
28: slider
31: outer sheath
32: inner sheath
33: attachment/detachment member
41: first tubular part
41A: outer peripheral side sliding part
42: second tubular part
42A: inner peripheral side sliding part
42C: grip member
43: coupling member
43A: groove
44: seal member
44A: inner peripheral surface
44B: protrusion
44C: outer peripheral surface
45A: small-diameter tube
45B: large-diameter tube
45C: grip member
46: fitting part
46A: tapered part
46B: circumferential part
47: third tubular part
47A: outer peripheral surface
51: first engaging part
51A: inner peripheral surface
51B: notch
51C: recess part
52: second engaging part
52A, 52B: clamping piece
60: lesion portion
71: endoscopic treatment tool device
72: oversheath
73: outer sheath
74: second tubular part
74A: inner peripheral side sliding part
75: seal member
75A: inner peripheral surface
75B: protrusion
75C: outer peripheral surface
81: endoscopic treatment tool device
82: oversheath
83: attachment/detachment member
84: engaging part
84A: inner peripheral surface
84B: notch
85: fitting part
91: attachment/detachment member
92: catheter device
93: guide catheter
94: guide catheter cock
94a: guide wire opening
94b: liquid feeding socket
95: guide wire
100: endoscope
101: insertion part
102: distal end part
103: image sensor
104: illumination optical system
105: distal end part body
106: cap
106A: peripheral surface part
106B: end surface part
106C: opening window
107: elevator
107A: rotational movement shaft
108: elevator operating wire
108: elevator operating wire
109: guide tube
110: treatment tool
110A: distal end part
111: attachment/detachment member
112: elevator wire operating part
112A: operation lever
113: signal cable
114: light guide
D: doctor
D 11: outer diameter
D21: inner diameter
D22: inner diameter
DH1, DH2: hand
H: assistant
L 11: length
L12: length
L13: length
M: mouth
MP: endoscopic mouthpiece
P: patient
R1: first resistance force
R2: second resistance force
X: attachment direction
Y: axial direction
Z: insertion direction
α, β: inclined angle

## Claims

1. An oversheath into which a flexible sheath of an endoscopic treatment tool is inserted and that is inserted into a forceps channel of an endoscope in cooperation with the endoscopic treatment tool, the oversheath comprising:
a first tubular part including an outer peripheral side sliding part that slides on an inner peripheral surface of a forceps port as an introduction port into the forceps channel; and
a second tubular part provided at a base end part of the first tubular part and including an inner peripheral side sliding part that allows the endoscopic treatment tool to move forward and backward with respect to the forceps channel.

2. The oversheath according to claim 1, further comprising:
an outer sheath in which the first tubular part and the second tubular part are provided integrally with each other; and
an inner sheath in which a fitting part that is externally fitted to the endoscopic treatment tool, and a third tubular part that slides on the inner peripheral side sliding part and covers at least a part of the flexible sheath are provided integrally with each other.

3. The oversheath according to claim 2, further comprising:
an attachment/detachment member provided integrally with the outer sheath and engaging with a forceps valve mounted on the forceps channel,
wherein, in a case in which the attachment/detachment member engages with the forceps valve, the outer sheath is attached to the endoscope via the attachment/detachment member.

4. The oversheath according to claim 3,
wherein the attachment/detachment member includes
a first engaging part that engages with the forceps valve, and
a second engaging part that engages with a part of the endoscope.

5. The oversheath according to claim 3 or 4,
wherein the attachment/detachment member is disposed in a direction in which an attachment direction to the forceps valve is orthogonal to an axial direction of the outer sheath or in a direction inclined from the direction orthogonal to the axial direction.

6. The oversheath according to any one of claims 2 to 5,
wherein the endoscopic treatment tool is movable forward and backward and rotatable with respect to the oversheath within a range in which the third tubular part and the inner peripheral side sliding part slide.

7. The oversheath according to any one of claims 2 to 6,
wherein the fitting part is fitted to the flexible sheath of the endoscopic treatment tool or an operating part installed consecutively with the flexible sheath.

8. The oversheath according to any one of claims 2 to 7,
wherein the third tubular part has a dimension in an axial direction longer than a range in which the endoscopic treatment tool moves forward and backward with respect to the forceps channel.

9. The oversheath according to any one of claims 2 to 8,
wherein the outer sheath includes a seal member that fills a gap between the outer sheath and the inner sheath at a base end part of the second tubular part.

10. The oversheath according to any one of claims 1 to 9,
wherein the first tubular part has a double-tube structure in which a length in an axial direction is expandable and contractible.

11. The oversheath according to any one of claims 1 to 10,
wherein a length of the first tubular part is shorter than a length of the flexible sheath.

12. The oversheath according to any one of claims 1 to 11,
wherein the forceps channel includes a first inner diameter part located on a forceps port side, and a second inner diameter part located on a distal end part side of the endoscope with respect to the first inner diameter part and having a smaller inner diameter than the first inner diameter part, and
an outer diameter of the first tubular part is smaller than the first inner diameter part and is larger than the second inner diameter part.

13. The oversheath according to any one of claims 1 to 12,
wherein a first resistance force in a case of allowing the first tubular part to move forward and backward with respect to the forceps channel is larger than a second resistance force in a case of allowing the endoscopic treatment tool to move forward and backward with respect to the second tubular part.

14. An endoscopic treatment tool device comprising:
a treatment tool body including a tubular flexible sheath, an operating wire inserted into the flexible sheath, and an operating part provided at a base end part of the flexible sheath; and
an oversheath having an insertion pipe line into which the flexible sheath is inserted and being inserted into a forceps channel of an endoscope in cooperation with the treatment tool body,
wherein the oversheath includes
an outer sheath in which a first tubular part including an outer peripheral side sliding part that slides on an inner peripheral surface of a forceps port as an introduction port into the forceps channel, and a second tubular part provided at a base end part of the first tubular part and including an inner peripheral side sliding part that allows the treatment tool body to move forward and backward with respect to the forceps channel are provided integrally with each other, and
an inner sheath in which a fitting part that is externally fitted to the treatment tool body, and a third tubular part that slides on the inner peripheral side sliding part and covers at least a part of the flexible sheath are provided integrally with each other.

15. The endoscopic treatment tool device according to claim 14, further comprising:
an attachment/detachment member provided integrally with the outer sheath and engaging with a forceps valve mounted on the forceps channel,
wherein, in a case in which the attachment/detachment member engages with the forceps valve, the outer sheath is attached to the endoscope via the attachment/detachment member.

16. An endoscopic instrument comprising:
an attachment/detachment member engaging with a forceps valve mounted on a forceps channel of an endoscope,
wherein the attachment/detachment member includes
a first engaging part that engages with the forceps valve, and
a second engaging part that engages with a part of the endoscope, and
in a case in which the attachment/detachment member engages with the forceps valve, the endoscopic instrument is attached to the endoscope via the attachment/detachment member.

17. The endoscopic instrument according to claim 16, further comprising:
a treatment tool operating part that operates an endoscopic treatment tool,
wherein the attachment/detachment member is provided integrally with the treatment tool operating part.

18. The endoscopic instrument according to claim 16,
wherein the part of the endoscope engaged with the second engaging part is located on a proximal end side of the forceps valve.

19. The endoscopic instrument according to claim 16,
wherein the endoscopic instrument is disposed at a position at which an axial direction intersects with an attachment/detachment direction of the attachment/detachment member.

20. The endoscopic instrument according to claim 16,
wherein the part of the endoscope engaged with the second engaging part is a treatment tool introduction part that constitutes an operating part of the endoscope, and
the endoscopic instrument is disposed in a direction along the treatment tool introduction part.
